(19) Europäisches Patentamt
European Patent Office
Office européen des brevets

(11) Publication number: 0 307 077 A1

# (12) EUROPEAN PATENT APPLICATION

(21) Application number: 88306563.3

(22) Date of filing: 18.07.88

(51) Int. Cl.4: A61K 31/40 , //A61K37/02, C07D209/86,C07D403/06

The title of the invention has been amended (Guidelines for Examination in the EPO, A-III, 7.3).

(30) Priority: 21.07.87 US 76093

(43) Date of publication of application: 15.03.89 Bulletin 89/11

(84) Designated Contracting States: CH DE FR GB IT LI NL

(71) Applicant: MERCK FROSST CANADA INC.
16711 Trans-Canada Highway
Kirkland Quebec(CA)

(72) Inventor: Ford-Hutchinson, Anthony W.
69 Hyde Park
Beaconsfield, QUE H9W 5L7(CA)

(74) Representative: Hesketh, Alan, Dr. et al
European Patent Department Merck & Co., Inc. Terlings Park Eastwick Road
Harlow Essex, CM20 2QR(GB)

## (54) Tetrahydrocarbazoles for the improvement of cyclosporin therapy.

(57) Tetrahydrocarbazoles, especially Tetrahydrocarbazole-1-alkanoic acids are disclosed. The compounds are useful for improving cyclosporine therapy. In particular, the compounds are useful for limiting cyclosporine induced nephrotoxicity which comprises the adjunct administration in a mammal of an effective amount of cyclosporine and an effective amount of the tetrahydrocarbazole 1-alkanoic acids.

EP 0 307 077 A1

# TETRAHYDROCARBAZOLE 1-ALKANOIC ACIDS FOR THE IMPROVEMENT OF CYCLOSPORINE THERAPY

## BACKGROUND OF THE INVENTION

This invention relates to terrahydrocarbazole 1-alkanoic acids for improving cyclosporine therapy. In particular, the compounds are useful for limiting cyclosporine-induced nephrotoxicity which comprises the adjunct administration in a mammal of an effective amount of cyclosporine and an effective amount of the tetrahydrocarbazole 1-alkanoic acids.

The cyclosporines comprise a class of structurally distinct, cyclic, poly-N-methylated undecapeptides having valuable pharmacological, in particular immunosuppressive, anti-inflammatory and anti-protozoal activity. The first of the cyclosporines to be isolated and the "parent" compound of the class, is the naturally occurring fungal metabolite known as cyclosporine A, the production and properties of which are described e.g. in U.S Patent 4,117,118. Since the original discovery of cyclosporine A, a wide variety of naturally occurring cyclosporines have been isolated and identified and many further non-natural cyclosporines have been prepared by synthetic or semi-synthetic means or by the application of modified culture techniques. The class comprised by the cyclosporines is thus now substantial and includes, for example, the naturally occurring cyclosporines ($Thr^2$)-, ($Val^2$)- and ($Nva^2$)-cyclosporine (also known as cyclosporines C, D and G, respectively), as well as various semi-synthetic derivatives thereof, such as their dihydro derivatives (e.g. as disclosed in U.S. Patents 4,108,985; 4,210,581 and 4,220,641) including e.g. (Dihydro-$MeBmt^1$)-($Val^2$)-cyclosporine (also known as dihydrocyclosporine D) and other natural and artificial cyclosporines such as those disclosed in European Patent Publication no. 0,058,134 B1, for example [(D)-$Ser^8$]-cyclosporine.

In accordance with now conventional nomenclature for the cyclosporines, these are defined herein by reference to the structure of cyclosporine A. This is done by first indicating those residues in the molecule which differ from those present in cyclosporine A and then applying the term "cyclosporine" to characterize the remaining residues which are identical to those present in cyclosporine A. Cyclosporine A has the formula I:

| A-B-Sar-MeLeu-Val-MeLeu-Ala-(D)Ala-MeLeu-MeLeu-MeVal | |
|---|---|
| 1 2 3 4 5 6 7 8 9 10 11 | (I) |

wherein A represents the -MeBmt- [N-methyl-(4R)-4-but-2E-en-1-yl-4-methyl-(L)threonyl] residue of formula II

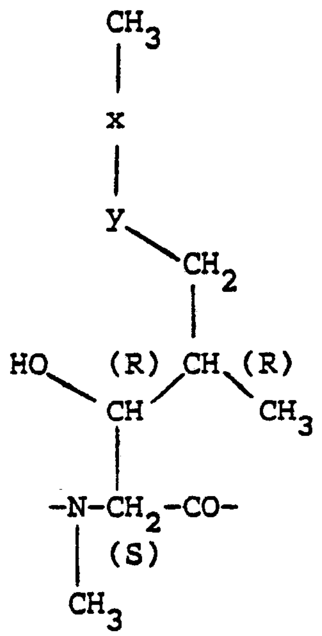

(II)

in which -x-y- is -CH=CH- (trans), and B is - a -Abu-. Accordingly, (Thr[2])-cyclosporine (cyclosporine C) is the compound of formula I, wherein A has the meaning given above and B is -Thr-, and (Dihydro-MeBmt[1])-(Val[2])-cyclosporine (dihydrocyclosporine D) is the compound of formula I, wherein A represents the -dihydro-MeBmt- residue of formula II above in which -x-y- is $-CH_2CH_2-$, and B is -Val-.

As the "parent" compound of the class, cyclosporine A has so far received the most attention. The primary area of clinical investigation for cyclosporine A has been as an immunosuppressive agent, in particular in relation to its application to recipients of organ transplants, e.g. heart, lung, combined heart-lung, liver, kidney, spleen, bone marrow, skin and corneal transplants and, in particular, allogenic organ transplants. In this field cyclosporine A has achieved a remarkable success and reputation and is now commercially available and widely employed in clinic.

At the same time, applicability of cyclosporine A to various diseases thought to have an autoimmune basis (including for example: multiple sclerosis, Guillan-Barre syndrome, uveitis, myasthenia gravis, Heymann nephritis, juvenile diabetes type I, systemic lupus erythematosus, aplastic anaemia, pure red cell anaemia, idiopathic thrombocytopaenia, polychondritis, sclerodoma, Wegener granulamatosis, dermatomyositis, chronic active hepatitis, auto-immune male infertility, psoriasis and psoriatic arthritis, Steven-Johnson syndrome, idiopathic sprue, Chrone's disease, sarcoidosis, glomerulonephritis, interstitial lung fibrosis and primary billiary cirrhosis) and to inflammatory conditions, in particular inflammatory conditions with an aetiology including an auto-immune component such as arthritis and rheumatic diseases, has been intensive and reports and results in vitro, in animal models and in clinical trials are widespread in the literature.

A further area of investigation of cyclosporines has been the potential applicability as an anti-parasitic, in particular anti-protozoal agent, with possible uses suggested including malaria treatment and treatment of schistosomiasis, filariasis, leishmania and coccidiomycosis.

Other cyclosporines exhibit the same overall pharmacological utility as cyclosporine A and various proposals for application, in particular in one or other of the above identified indications, are prevelant in the literature.

Despite the very major contribution which cyclosporine A has made to the art, in particular in the field of transplant surgery, and despite cyclosporine A's wide acceptance and success in the clinic, an obvious negative feature has been its reported nephrotoxicity. A principal limitation to the use of cyclosporine A in man is the induction of nephrotoxicity characterized by reversible increases in blood urea nitrogen and serum creatinine levels and a depression of creatinine clearance (Bennett and Pultiam, Ann. Intern. Med. 99, 851-854; 1983). Models of cyclosporine A-induced nephrotoxicity have been developed in rats and prolonged treatment results in stimulation of the renin angiotensin accompained by a progressive increase in glomerular synthesis and urinary excretion of thrombxane $A_2$ (Penico et al, J. Pharm. Exp. Therap. 239, 229-235, 1986). In a subsequent study, the authors showed administration of a thromboxane synthetase inhibitor (Uk-38,485) reduced urinary $TxB_2$ and increased the glomerular filtration rate (Penico et al., Am. J. Physiol 252, F581-F587, 1986).

A means of reducing the side effect of nephrotoxicity would clearly be of major benefit. In accordance with the present invention, it has now been found that cyclosporine-induced nephrotoxicity, in particular cyclosporine A, can be limited by conjugate administration of a tetrahydro-carbazole 1-alkanoic acid of the present invention.

DESCRIPTION OF THE INVENTION

The present invention relates to a method for limiting cyclosporine induced nephrotoxicity which comprises the adjunct administration in a mammal of an effective amount of cyclosporine and an effective amount of a compound of Formula I:

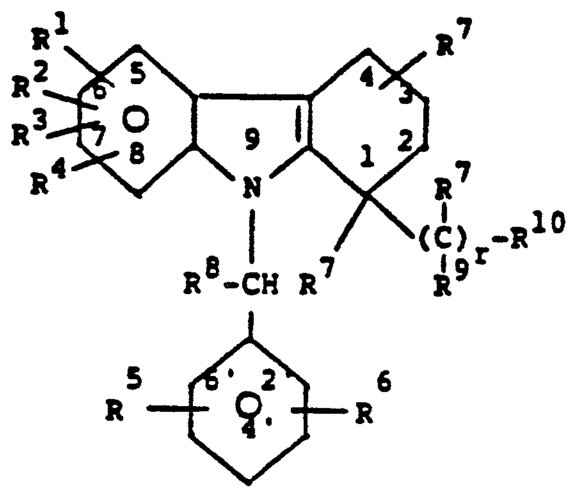

I

wherein:

$R^1$, $R^2$, $R^3$, $R^4$, $R^5$ and $R^6$ are each independently selected from:

(1) hydrogen;

(2) alkyl having 1 to 6 carbon atoms;

(3) alkenyl having 2 to 6 carbon atoms;

(4) $-(CH_2)_nM$

wherein n is 0 to 3 and M is

a) $OR^{13}$;

b) halogen;

c) $CF_3$;

d) $SR^{13}$;

e) phenyl or substituted phenyl wherein substituted phenyl is as defined below in the definition of $R^{13}$;

f) $COOR^{14}$;

g) $\overset{\overset{O}{\|}}{C}-R^{15}$;

h) tetrazole;

i) $-NH-\overset{\overset{O}{\|}}{C}-R^{16}$ wherein $R^{16}$ is $C_1$ to $C_6$ alkyl, benzyl or phenyl;

j) $-NR^{14}R^{14}$;

k) $-NHSO_2R^{17}$ wherein $R^{17}$ is $C_1$ to $C_6$ alkyl, 4-methylphenyl, phenyl, or $CF_3$;

l) $-\overset{\overset{O}{\|}}{C}-CH_2OH$;

m) $-SOR^{13}$;

n) $-CONR^{14}R^{14}$;

o) $-SO_2NR^{14}R^{14}$;

p) $-SO_2R^{13}$;

q) $NO_2$;

r) 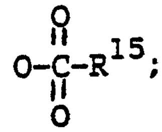

s) $O-C-NR^{14}R^{14}$;

t) 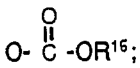

v) $N_3$;

u) CN;

$R^7$ is H or alkyl of 1 to 6 carbons;

$R^8$ is H or alkyl of 1 to 6 carbon atoms;

each $R^9$ is independently H, OH, $C_1$ to $C_4$-O-alkyl or alkyl of 1 to 4 carbons;

$R^{10}$ is COOH; $CH_2OH$; CHO; tetrazole; $NHSO_2R^{11}$ wherein $R^{11}$ is OH, alkyl or alkoxy of 1 to 6 carbons, perhaloalkyl of 1 to 6 carbons, phenyl or phenyl substituted by alkyl or alkoxy groups of 1 to 3 carbons, halogen, hydroxy, COOH, CN, formyl or acyl to 1 to 6 carbons; $CONHSO_2R^{11}$; hydroxymethylketone; CN; or $CON(R^9)_2$;

r is 1 to 6;

each $R^{13}$ independently is H; $C_1$ to $C_6$ alkyl; benzyl; phenyl or substituted phenyl wherein the substituents are $C_1$ to $C_3$ alkyl, halogen, CN, $CF_3$, $COOR^{14}$, $CH_2COOR^{14}$, $C_1$ to $C_3$ alkoxy, or $C_1$ to $C_4$ perfluoroalkyl;

each $R^{14}$ is independently H, phenyl, benzyl or $C_1$ to $C_6$ alkyl; and,

each $R^{15}$ independently is H, $(CH_2)_mCOOR^{14}$ wherein m is 0 to 4, $C_1$ to $C_6$ alkyl, $CF_3$, phenyl, or substituted phenyl wherein substituted phenyl is as defined above in the definition of $R^{13}$;

or a pharmaceutically acceptable salt thereof.

As used herein, the terms "each independently" or the equivalents thereof are employed to describe a number of possible position isomers and/or structural variations. For example, as described above, the following unit is attached to position 1 of the tetrahydrocarbazole ring:

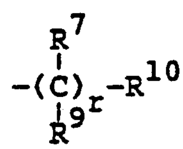

The letter r represents possible alkane chains of from 1 to 6 carbon atoms, each having the $R^7$ and $R^9$ substituent groups. On each carbon atom of the alkane chain, the $R^7$ and/or $R^9$ substituent may be different. The above description therefore contemplates structures such as the following for the segment --$(CR^7R^9)_r$-:

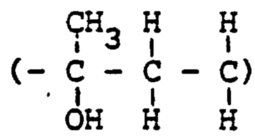 , $(-CH_2-)$

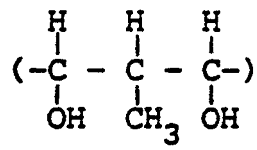 , 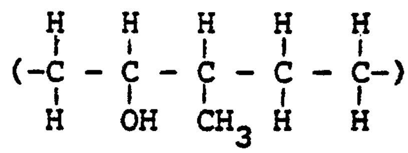 ,

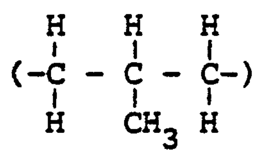 , and the like.

If an $R^9$ is OH and $R^{10}$ is $CO_2H$, such compounds may form a lactone, and such lactones are to be regarded as part of the present invention.

The alkyl groups referred to above may be straight chain or branched or may include cycloalkyl groups. As used herein, the term "lower" as applied to alkyl, acyl, alkoxy and the like, unless stated otherwise refers to groups having 1 to 6 carbon atoms. Halogen or halo means fluoro, chloro, bromo and/or iodo.

Pharmaceutically acceptable salts of the compounds described herein are included within the scope of the present invention. Such salts may be prepared from pharmaceutically acceptable non-toxic bases including inorganic bases and organic bases. Salts derived from inorganic bases include sodium, potassium, lithium, ammonium, calcium, magnesium, ferrous, zinc, copper, manganous, aluminum, ferric, manganic salts and the like. Particularly preferred are the potassium, sodium, calcium and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethanolamine, diethylamine, trithylamine, tripropylamine, ethanolamine, 2-dimethylaminoethanol, 2-diethylamino-ethanol, tomethamine, lysine, arginine, histidine, caffeine, procaine, hydrabamine, choline, imidazole, betaine, ethylenediamine, glucosamine, methylglucamine, theobromine, purines piperazine, N,N-dibenzyl-ethylenediamine, piperidine, N-ethyl-piperidine, morpholine, N-ethylmorpholine, polyamine resins and the like.

The method of the invention can be utilized with any use of cyclosporine therapy. For example, the method can be utilized in all of the uses described hereinabove, with organ transplants being the most prevalent use.

The preferred cyclosporine of the invention is cyclosporine A.

As previously indicated, the method of the invention is particularly applicable to limiting cyclosporine-induced nephrotoxicity. Kidney damage, e.g. functional changes such as appearance of proteinuria, changes in renal blood flow and glomerular filtration rate and changes in electrolyte excretion and inulin clearance.

In accordance with the method of the present invention, cyclosporine and a tetrahydrocarbazole 1-alkanoic acid can be administered separately at different times during the course of therapy or substantially concommitantly. Thus treatment with the tetra hydrocarbazole 1-alkanoic acid can commence prior to cyclosporine treatment, or subsequent to commencement of cyclosporine treatment. The present invention is to be understood as embracing all such regimes of treatment and the term "adjunct administration" is to be interpreted accordingly.

Doses of cyclosporine to be administered in practicing the method of the invention will of course vary depending upon, e.g. the particular cyclosporine employed, the mode of administration, the condition to be treated (e.g. whether treatment is for the purposes of immunosuppression or otherwise, and if for immunosuppression whether for use in relation to e.g. organ transplant, bone-marrow transplant, or the treatment of auto-immune disease), as well as the effect desired. In addition, dosaging will generally require adjustment for individual patients in order to establish an appropriate long-term drug serum concentration, e.g. by administration of an initial daily starting or "loading" dose with subsequent dose adjustment (generally dose reduction) in accordance with serum levels, e.g. as determined by regular radioimmunoassay (RIA) monitoring.

In general, amounts administered will be of the same order to those conventionally employed in cyclosporine therapy, e.g. cyclosporine A therapy, i.e. required to achieve (i) immunosuppressive, (ii) anti-inflammatory, or (iii) anti-parasitic effectiveness. Thus, in general, satisfactory results are obtained on administration in a dose range of from about 1 or about 5 to about 50 mg/kg/day (e.g., in the case of cyclosporine A, from about 5 or about 10 to about 20 mg/kg/day during the initial phase of therapy, reducing to a maintenance dose of from about 1 or about 5 to about 10 mg/kg/day) administered to the patient orally, once or in divided doses 2 or 3x a day. Where I.V. administration is required, e.g. administration by infusion (for example in the initial phase of treatment) lower dosages, e.g. of the order of from about 0.5 or about 1 to about 10 (e.g. in the case of cyclosporine A, from about 1 or about 3 to about 5 mg/kg/day for an initiating dose, or to about 2.5 mg/kg/day for a maintenance dose) are generally indicated.

Preferred compounds that are useful in the present invention comprise the compounds of formula I wherein:

$R^1$, $R^2$, $R^3$, $R^4$, $R^5$ and $R^6$ are each independently selected from:

(1) hydrogen;
(2) alkyl having 1 to 6 carbon atoms;
(3) alkenyl having 2 to 6 carbon atoms;
(4) $-(CH_2)_nM$

wherein n is 0 or 1 and M is as defined previously for Formula I;

$R^{10}$ is COOH; $CH_2OH$; CHO; tetrazole; $CONHSO_2R^{11}$ wherein $R^{11}$ is OH, alkyl or alkoxy of 1 to 6 carbons, perhaloalkyl of 1 to 6 carbons, phenyl or phenyl substituted by alkyl or alkoxy groups of 1 to 3 carbons, halogen, hydroxy, COOH, CN, formyl or acyl to 1 to 6 carbons; hydroxymethylketone; CN; or $CON(R^9)_2$;

r is 1 to 6; and the remaining substituents are as defined previously for Formula I.

More preferred compounds of the present invention comprise the compounds of Formula I. wherein:

$R^1$, $R^2$, $R^3$, $R^4$, $R^5$ and $R^6$ are each independently selected from:

(1) hydrogen;
(2) alkyl having 1 to 6 carbon atoms;
(3) alkenyl having 2 to 6 carbon atoms;
(4) M wherein M is as defined initially for Formula I;

$R^{10}$ is COOH; $CH_2OH$; CHO; tetrazole; hydroxymethylketone;

r is 1 or 2; and the remaining substituents are as defined initially for Formula I.

Most preferred compounds of the present invention comprise the compounds of Formula I. wherein:

$R^1$, $R^2$, $R^3$, $R^4$, $R^5$ and $R^6$ are each independently selected from:

(1) hydrogen;
(2) alkyl having 1 to 6 carbon atoms;
(3) M wherein M is

a) $OR^{13}$;
b) halogen;
c) $CF_3$;
d) $SR^{13}$;
e) $COOR^{14}$;
f) $\overset{O}{\overset{\|}{C}}-R^{15}$;
g) tetrazole;
h) $-SOR^{13}$;
i) $-CONR^{14}R^{14}$;
j) $-SO_2NR^{14}R^{14}$;
k) $-SO_2R^{13}$;
l) $O-\overset{O}{\overset{\|}{C}}-R^{15}$;
m) CN;
n) $N_3$;

each $R^9$ is independently H, or alkyl of 1 to 4 carbons;

$R^{10}$ is COOH; or tetrazole,

r is 1 and the remaining substituents are as defined initially for Formula I.

In the above most preferred embodiment, those compounds are particularly preferred wherein at least one of $R^1$ to $R^4$ is not hydrogen; one of $R^5$ or $R^6$ is not hydrogen; $R^7$ is hydrogen; $R^9$ is hydrogen, and the remaining substituents are as defined in the most preferred embodiment.

A further embodiment of the present invention are the following novel compounds (Table 1). Among the resolved isomers in Table 1, the (-) isomers, compounds 3,37,39 and 41, are preferred.

**Table 1**
**Tetrahydrocarbazole**
**Alkanoic Acids**

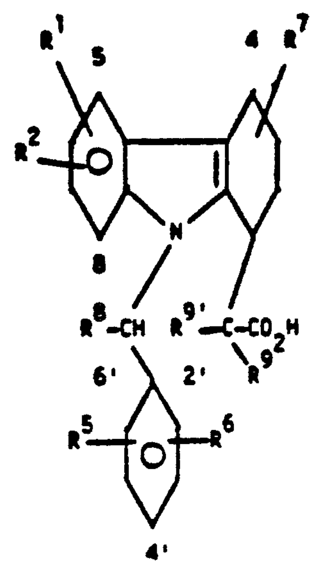

| Compound | $R^1$ | $R^2$ | $R^5$ | $R^6$ | $R^9$, $R^{9'}$ | $R^7$ | $R^8$ |
|---|---|---|---|---|---|---|---|
| 1 (Ex.1) | 6-F | H | 4'-Cl | H | H, H | H | H |
| 2 (Ex. 4) | 6-OMe | H | 4'-Cl | H | H, H | H | H |
| 3 (Ex. 7) | 6-F (-) isomer | H | 4'-Cl | H | H, H | H | H |
| 4 (Ex. 8) | 6-F (+) isomer | H | 4'-Cl | H | H, H | H | H |
| 5 (Ex. 9) | 6-F | H | H | H | H, H | H | H |
| 6 (Ex. 10) | 6-F | H | 4'-OMe | H | H, H | H | H |

| Compound | $R^1$ | $R^2$ | $R^5$ | $R^6$ | $R^9$, $R^{9'}$ | $R^7$ | $R^8$ |
|---|---|---|---|---|---|---|---|
| 7 (Ex. 11) | 6-F | H | 3'-Cl | 4'-Cl | H, H | H | H |
| 8 (Ex. 12) | 6-F | H | H | H | H, H | H | Me |
| 9 (Ex 13) | H | H | 4'-Cl | H | H, H | H | H |
| 10 (Ex. 14) | 6-Cl | H | 4'-Cl | H | H, H | H | H |
| 11 (Ex. 15) | 8-Me | H | 4'-Cl | H | H, H | H | H |
| 12 (Ex. 16) | 6-Br | H | 4'-Cl | H | H, H | H | H |
| 13 (Ex. 17) | 6-Me | H | 4'-Cl | H | H, H | H | H |
| 14 (Ex. 19) | 8-F | H | 4'-Cl | H | H, H | H | H |
| 15 (Ex. 20) | 6-F | H | 4'-Cl | H | H, H | 3-t-Bu | H |
| 16 (Ex. 21) | 5-F | H | 4'-Cl | H | H, H | H | H |
| 17 (Ex. 21) | 7-F | H | 4'-Cl | H | H, H | H | H |
| 18 (Ex. 22) | 5-Cl | 7-Cl | 4'-Cl | H | H, H | H | H |
| 19 (Ex. 23) | 6-Cl | 8-Cl | 4'-Cl | H | H, H | H | H |
| 20 (Ex. 18) | 6-F | H | 4'-Cl | H | Me, H | H | H |
| 21 | 6-F | H | 4'-Cl | H | Me, Me | H | H |

| Compound | $R^1$ | $R^2$ | $R^5$ | $R^6$ | $R^9$, $R^{9'}$ | $R^7$ | $R^8$ |
|---|---|---|---|---|---|---|---|
| 22 | 6-F | H | 4'-Cl | H | H, H | 1-Me | H |
| 23 | 8-Br | H | 4'-Cl | H | H,H | H | H |
| 24 (Ex. 24) | 6-CH(Me)$_2$ | H | 4'-Cl | H | H,H | H | H |
| 25 (Ex. 25) | 6-C(Me)$_3$ | H | 4'-Cl | H | H,H | H | H |
| 26 (Ex. 26) | 6-CF$_3$ | H | 4'-Cl | H | H,H | H | H |
| 27 (Ex. 27) | 6-SMe | H | 4'-Cl | H | H,H | H | H |
| 28 (Ex. 28) | 6-S(0)Me | H | 4'-Cl | H | H,H | H | H |
| 29 (Ex. 29) | 6-S(0)$_2$Me | H | 4'-Cl | H | H,H | H | H |
| 30 (Ex. 30) | 8-CH(Me)$_2$ | H | 4'-Cl | H | H,H | H | H |
| 31 (Ex. 31) | 8-SMe | H | 4'-Cl | H | H,H | H | H |
| 32 (Ex. 32) | 8-S(0)Me | H | 4'-Cl | H | H,H | H | H |
| 33 (Ex. 33) | 6-F | H | 4'-Cl | H | H,H | 3-Me | H |
| 34 (Ex. 34) | 6-F | 8-F | 4'-Cl | H | H,H | H | H |
| 35 (Ex. 35) | 6-Me | 8-Me | 4'-Cl | H | H,H | H | H |

| Compound | $R^1$ | $R^2$ | $R^5$ | $R^6$ | $R^9$, $R^{9'}$ | $R^7$ | $R^8$ |
|---|---|---|---|---|---|---|---|
| 36 (Ex. 36) | 6-OMe | 8-Me | 4'-Cl | H | H,H | H | H |
| 37 (Ex. 37) | 6-F(-)Isomer | 8-F | 4'-Cl | H | H,H | H | H |
| 38 (Ex. 38) | 6-F(+)Isomer | 8-F | 4'-Cl | H | H,H | H | H |
| 39 (Ex. 39) | 8-Me(-)Isomer | H | 4'-Cl | H | H,H | H | H |
| 40 (Ex. 40) | 8-Me(+)Isomer | H | 4'-Cl | H | H,H | H | H |
| 41 (Ex. 41) | 8-F(-)Isomer | H | 4'-Cl | H | H,H | H | H |
| 42 (Ex. 42) | 8-F(+)Isomer | H | 4'-Cl | H | H,H | H | H |
| 43 | 6-F | 8-F | 3'-Cl | 4'-Cl | H,H | H | H |
| 44 (Ex. 46) | 6-F | 8-F | 2'-Cl | 4'-Cl | H,H | H | H |
| 45 | 6-F | 8-F | 4'-OMe | H | H,H | H | H |
| 46 | 6-F | 8-F | 4'-OH | H | H,H | H | H |
| 47 (Ex. 47) | 6-F | 8-F | 4'-SMe | H | H,H | H | H |
| 48 | 6-F | H | 4'-S(O)Me | H | H,H | H | H |
| 49 (Ex. 59) | 6-F | 8-F | 4'-NHCOMe | H | H,H | H | H |
| 50 | 6-F | H | 4'-$S(O)_2Me$ | H | H,H | H | H |

| Compound | $R^1$ | $R^2$ | $R^5$ | $R^6$ | $R^9$, $R^{9'}$ | $R^7$ | $R^8$ |
|---|---|---|---|---|---|---|---|
| 51 | 6-F | H | 4'-F | H | H,H | H | H |
| 52 | 6-F | H | 4'-Br | H | H,H | H | H |
| 53 | 6-F | 8-Me | 4'-Cl | H | H,H | H | H |
| 54 | 6-F | H | 4'-$CO_2H$ | H | H,H | H | H |
| 55 | 6-F | H | 4'-$CO_2Me$ | H | H,H | H | H |
| 56 | 6-F | 8-F | 4'-n-$C_3H_7$ | H | H,H | H | H |
| 57 | 6-F | 8-F | 3'-I | 4'-OH | H,H | H | H |
| 58 | 6-F | 8-F | 4'-I | H | H,H | H | H |
| 59 | 6-$N_3$ | H | 4'-Cl | H | H,H | H | H |
| 60 | 6-F | H | 4'-$N_3$ | H | H,H | H | H |
| 61 (Ex. 48) | 6-F | 8-F | 4'-S(0)Me | H | H,H | H | H |
| 62 (Ex. 49) | 6-F | 8-F | 4'-$S(0)_2Me$ | H | H,H | H | H |
| 63 (Ex. 50) | 6-F(-)isomer | 8-F | 4'-$S(0)_2Me$ | H | H,H | H | H |
| 64 (Ex. 51) | 6-F(+)isomer | 8-F | 4'-$S(0)_2Me$ | H | H,H | H | H |
| 65 (Ex. 45) | 6-F | 8-F | 2'-Cl | H | H,H | H | H |

| Compound | $R^1$ | $R^2$ | $R^5$ | $R^6$ | $R^9$, $R^{9'}$ | $R^7$ | $R^8$ |
|---|---|---|---|---|---|---|---|
| 66 (Ex. 52) | 6-F | 8-F | 4'-$CF_3$ | H | H,H | H | H |
| 67 (Ex. 53) | 6-F | 8-F | 4'-F | H | H,H | H | H |
| 68 (Ex. 54) | 6-F | 8-F | 3'-Cl | H | H,H | H | H |
| 69 (Ex. 55) | 6-F | 8-F | 4'-$CO_2Me$ | H | H,H | H | H |
| 70 (Ex. 56) | 6-F | 8-F | 4'-$CONMe_2$ | H | H,H | H | H |
| 71 (Ex. 57) | 6-F | 8-F | 4'-COMe | H | H,H | H | H |
| 72 (Ex. 58) | 6-F | 8-F | 4'-$SO_2NMe_2$ | H | H,H | H | H |
| 73 (Ex. 60) | 6-F | 8-F | 4'-$NHSO_2Me$ | H | H,H | H | H |
| 74 (Ex. 61) | 6-F | 8-F | 4'-NHCONHMe | H | H,H | H | H |
| 75 (Ex. 62) | 6-F | 8-F | 4'-OMe | H | H,H | H | H |
| 76 | 6-F | 8-F | 4'-$CO_2H$ | H | H,H | H | H |
| 77 | 6-F | 8-F | 4'-$NH_2$ | H | H,H | H | H |
| 78 | 6-F | 8-F | 4'-OH | H | H,H | H | H |
| 79 | 6-F | 8-F | 4'-$OCH_2CO_2H$ | H | H,H | H | H |

The following reaction schemes illustrate the preparation of the compounds useful in the present invention:

Scheme I Preparation of Formula I Compounds

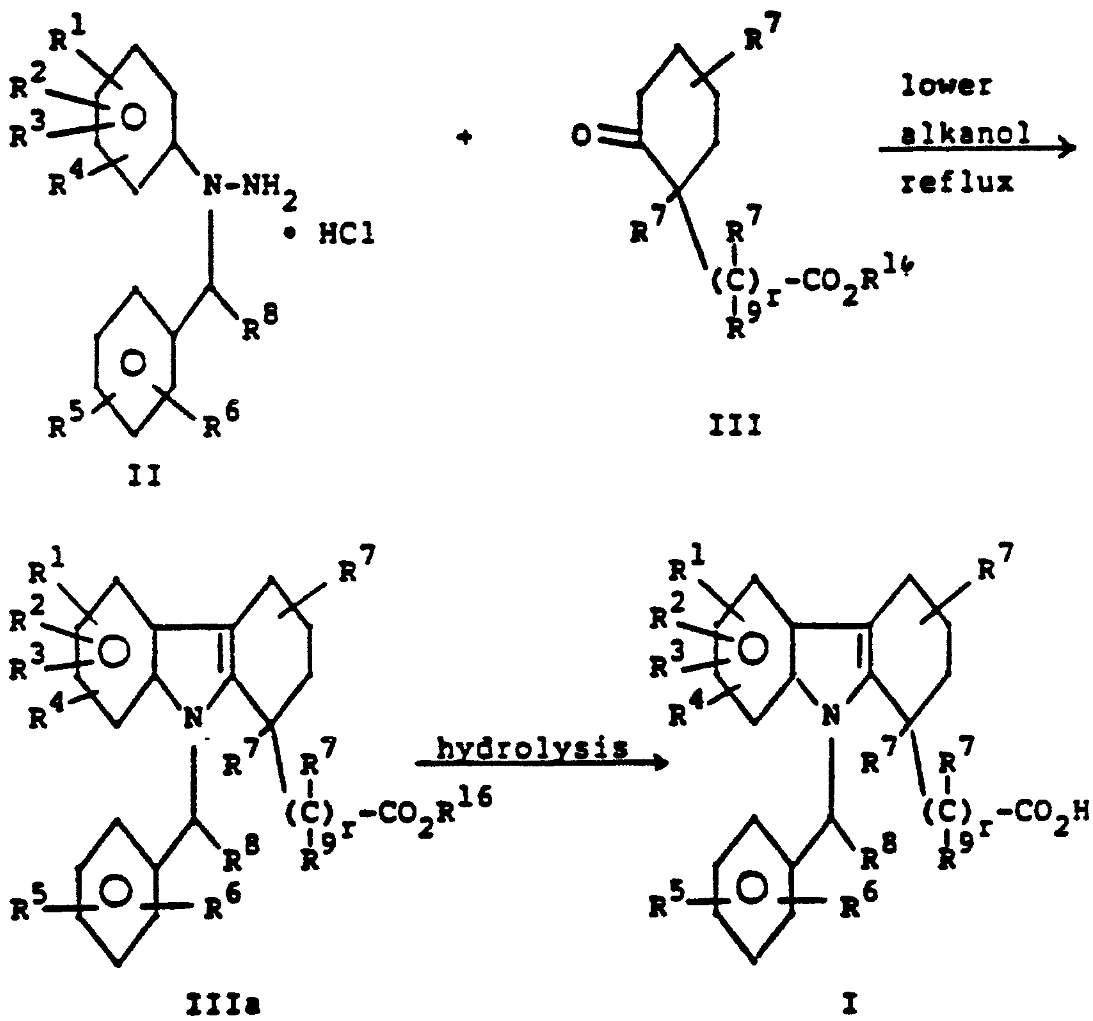


II III IIIa I

The reaction can be conveniently carried out in an alcohol solvent such as t-butanol, i-butanol, and the like. The hydrolysis of the ester intermediates IIIa is conveniently carried out by using NaOH or KOH in aqueous ethanol or methanol followed by acidification to obtain compounds of Formula I.

The following ketones (1,2,4) of structure III are known in the art, and ketones 3 and 5 are readily prepared by procedures analogous to those for the known ketones.

TABLE 2

ketones of Formula III

| No. | Structure | Reference |
| --- | --- | --- |
| 1. | 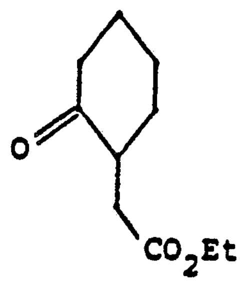 | Ethyl 2-cyclohexanone acetate; commercially available (Aldrich) |
| 2. | 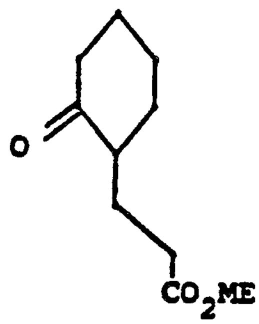 | Methyl 2-cyclohexanone propionate; J.A.C.S. 85 207 (1963) G. Stork, A. Brizzolara, H. Landesman, J. Scmuszkovicz and R. Terrell |
| 3. | 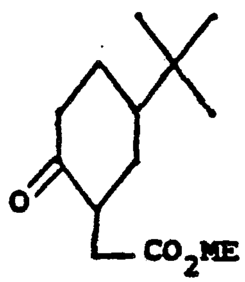 | Methyl 4-t-butyl-2-cyclohexanone acetate |

TABLE 2 (Cont'd)

4. 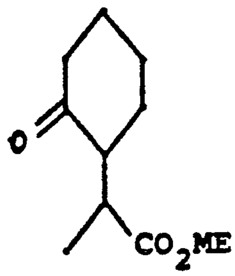

Methyl 2-(2-cyclohexanone) propionate
J.A.C.S. 85 207 (1963)
G. Stork, A. Brizzolara, H. Landesman, J. Scmuszkovicz and R. Terrell

5. 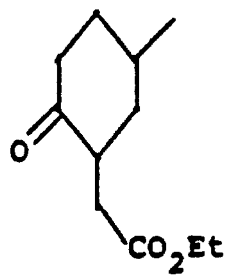

Ethyl 4-methyl-2-cyclohexanone acetate

The sequence described above is an application of the Fischer Indole Synthesis. Numerous-indole syntheses are described in reviews, such as, for example "Heterocyclic Compounds" Volume 25, Parts I, II, III, W. J. Houlihan (Ed.), Interscience, J. Wiley & Sons, N. Y., 1979. Appropriate manipulations of functional groups using sequences described in such reviews will lead to the compounds of the present invention.

Scheme II Preparation of Hydrazine Derivatives (II)

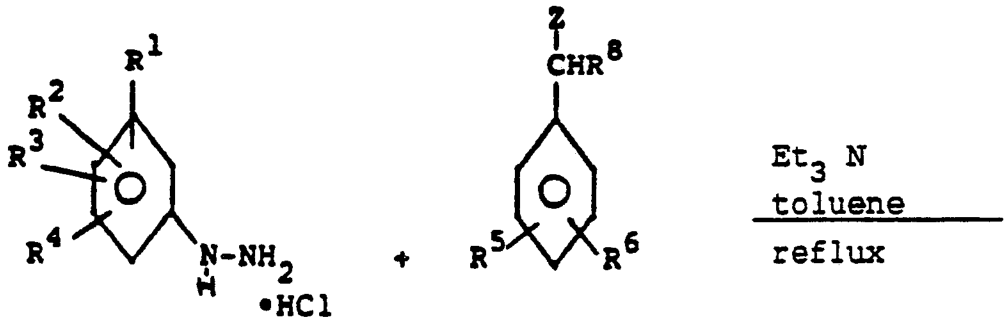


(IV) (V)

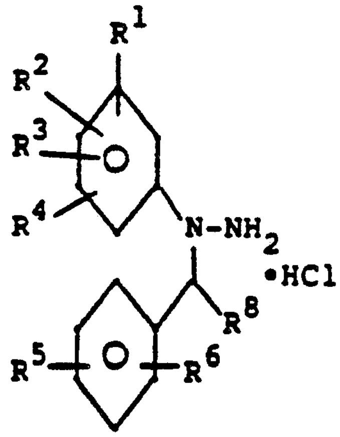

II

Z is a leaving group such as Cl, Br, I, mesylate or tosylate

With regard to Scheme II, the preparation of hydrazine starting materials is illustrated by the preparation of 1-(4 chlorobenzyl)-1-(4-methoxyphenyl)hydrazine. A mixture of 10 g of p-methoxyphenylhydrazine hydrochloride, 75 ml of toluene and 11.5 ml of triethylamine was heated at reflux for 60 minutes. Then, 7.1 g of p-chlorobenzyl chloride was added. After stirring 16 hours at reflux, triethylamine hydrochloride was filtered off and washed with ethyl ether. The filtrate and washing were concentrated in vacuo and chromatographed on a silica gel column (hexane-ethyl acetate, 9:1) to give 6.64 g of 1-(4-chlorobenzyl)-1-(4-methoxyphenyl)hydrazine. Other hydrazines, similarly prepared, are also shown in Table 3, below.

Examples of benzyl halides V are shown in Table 3b.

TABLE 3

Hydrazines

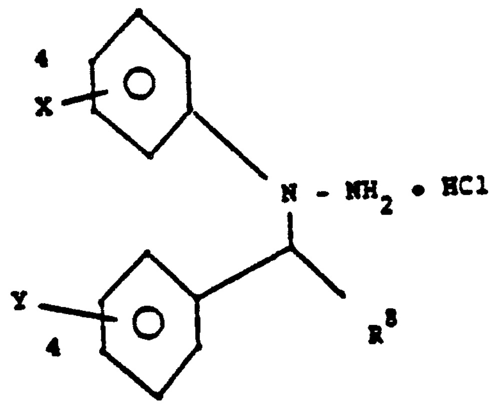

| Compound No. | X | Y | $R^8$ | Compound Name |
|---|---|---|---|---|
| 1. | 4-F | 4-Cl | H | 1-(4-chlorobenzyl)-1-(4-fluorophenyl) hydrazine hydrochloride |
| 2. | 3,5-$Cl_2$ | 4-Cl | H | 1-(4-chlorobenzyl)-1-(3,5-dichlorophenyl)hydrazine hydrochloride |

TABLE 3 (Cont'd)

| | | | | |
|---|---|---|---|---|
| 3. | 4-OMe | 4-Cl | H | 1-(4-chlorobenzyl)-1-(4-methoxyphenyl) hydrazine hydrochloride |
| 4. | 2-Me | 4-Cl | H | 1-(4-chlorobenzyl)-1-(2-methylphenyl) hydrazine hydrochloride |
| 5. | 4-Me | 4-Cl | H | 1-(4-chlorobenzyl)-1-(4-methylphenyl) hydrazine hydrochloride |
| 6. | 4-Cl | 4-Cl | H | 1-(4-chlorobenzyl)-1-(4-chlorophenyl) hydrazine hydrochloride |
| 7. | H | 4-Cl | H | 1-(4-chlorobenzyl)-1-(phenyl) hydrazine hydrochloride |
| 8. | 4-Br | 4-Cl | H | 1-(4-chlorobenzyl)-1-(4-bromophenyl) hydrazine hydrochloride |
| 9. | 3-F | 4-Cl | H | 1-(4-chlorobenzyl)-1-(3-fluorophenyl) hydrazine hydrochloride |
| 10. | 2,4-$Cl_2$ | 4-Cl | H | 1-(4-chlorobenzyl)-1-(2,4-dichlorophenyl)hydrazine hydrochloride |

TABLE 3 (Cont'd)

| | | | | |
|---|---|---|---|---|
| 11. | 4-F | H | H | 1-(benzyl)-1-(4-fluorophenyl) hydrazine hydrochloride |
| 12. | 4-F | 4-OMe | H | 1-(4-methoxybenzyl)-1-(4-fluorophenyl) hydrazine hydrochloride |
| 13. | 4-F | 3,4-$Cl_2$ | H | 1-(3,4-dichlorobenzyl)-1-(4-fluoro-phenyl) hydrazine hydrochloride. |
| 14. | 4-F | H | $CH_3$ | 1-[1-(phenyl)ethyl]-1-(4-fluorophenyl) hydrazine hydrochloride |
| 15. | 2-F | 4-Cl | H | 1-(4-chlorobenzyl)-1-(2-fluorophenyl) hydrazine hydrochloride. |
| 16. | 1-[2-(4-chlorophenyl)ethyl]-1-(4-fluorophenyl) hydrazine hydrochloride. | | | |
| 17. | 1-(2-propyl)-1-(4-fluorophenyl)hydrazine hydrochloride. | | | |
| 18. | 4-$CH(Me)_2$ | 4-Cl | H | 1-(4-chlorobenzyl)-1-(4-iso-propylphenyl)hydrazine hydrochloride |
| 19. | 4-$C(Me)_3$ | 4-Cl | H | 1-(4-chlorobenzyl)-1-(4-tert-butylphenyl)hydrazine)hydro-chloride |

TABLE 3 (Cont'd)

| | | | | |
|---|---|---|---|---|
| 20. | 4-$CF_3$ | 4-Cl | H | 1-(4-chlorobenzyl)-1-(4-trifluoromethylphenyl)-hydrazine hydrochloride |
| 21. | 4-SMe | 4-Cl | H | 1-(4-chlorobenzyl)-1-(4-methylthiophenyl)hydrazine hydrochloride |
| 22. | 2-CH(Me)$_2$ | 4-Cl | H | 1-(4-chlorobenzyl)-1-(2-isopropylphenyl)hydrazine hydrochloride |

Scheme III Alternative Preparation of Formula I Compounds

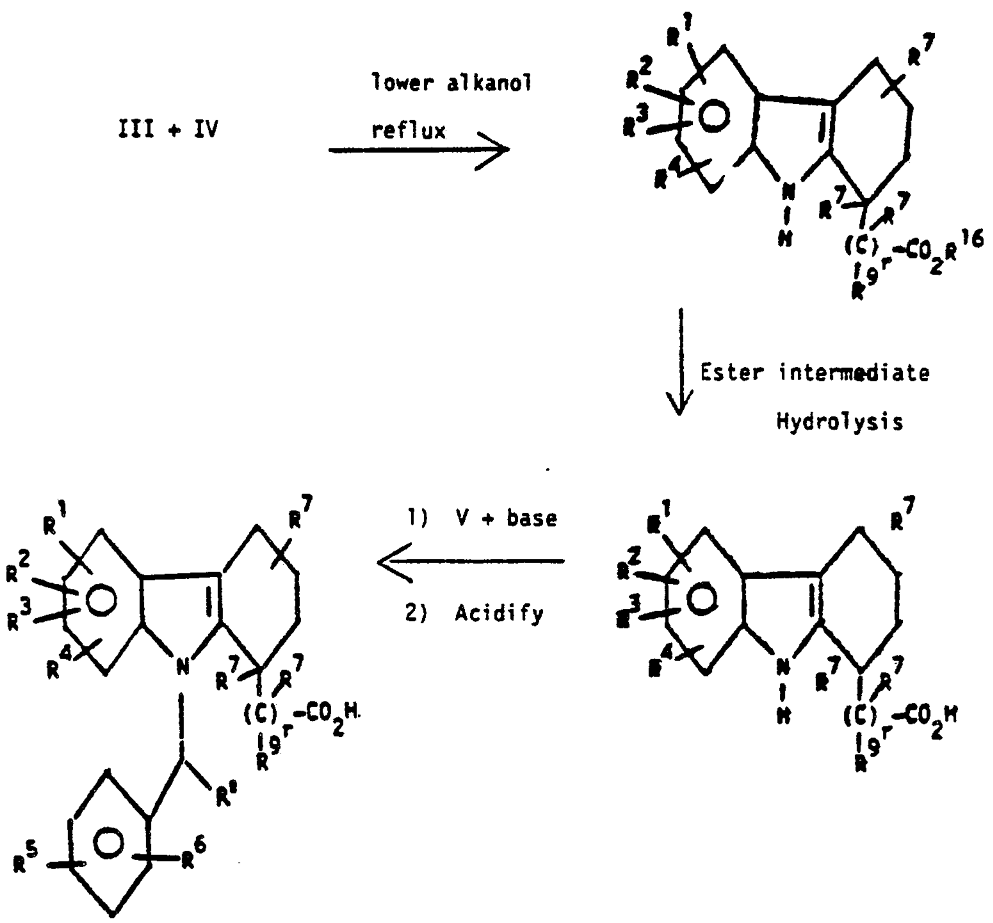


Acid intermediate

Scheme III illustrates an alternative synthesis of the compounds of Formula I. In this Scheme a Fischer

indole synthesis is carried out using a phenylhydrazine IV and the ketone III, followed by hydrolysis. The acid intermediate is then N-benzylated with the reagent V, preferably using a strong base such as potassium t-butoxide, sodium hydride (NaH) or potassium hexamethyldisilazide (KHMDS) to effect the reaction. Acidification of the reaction mixture then yields the free acid of I.

Scheme IV — Preparation of Sulfoxides and Sulfones of Formula I Compounds

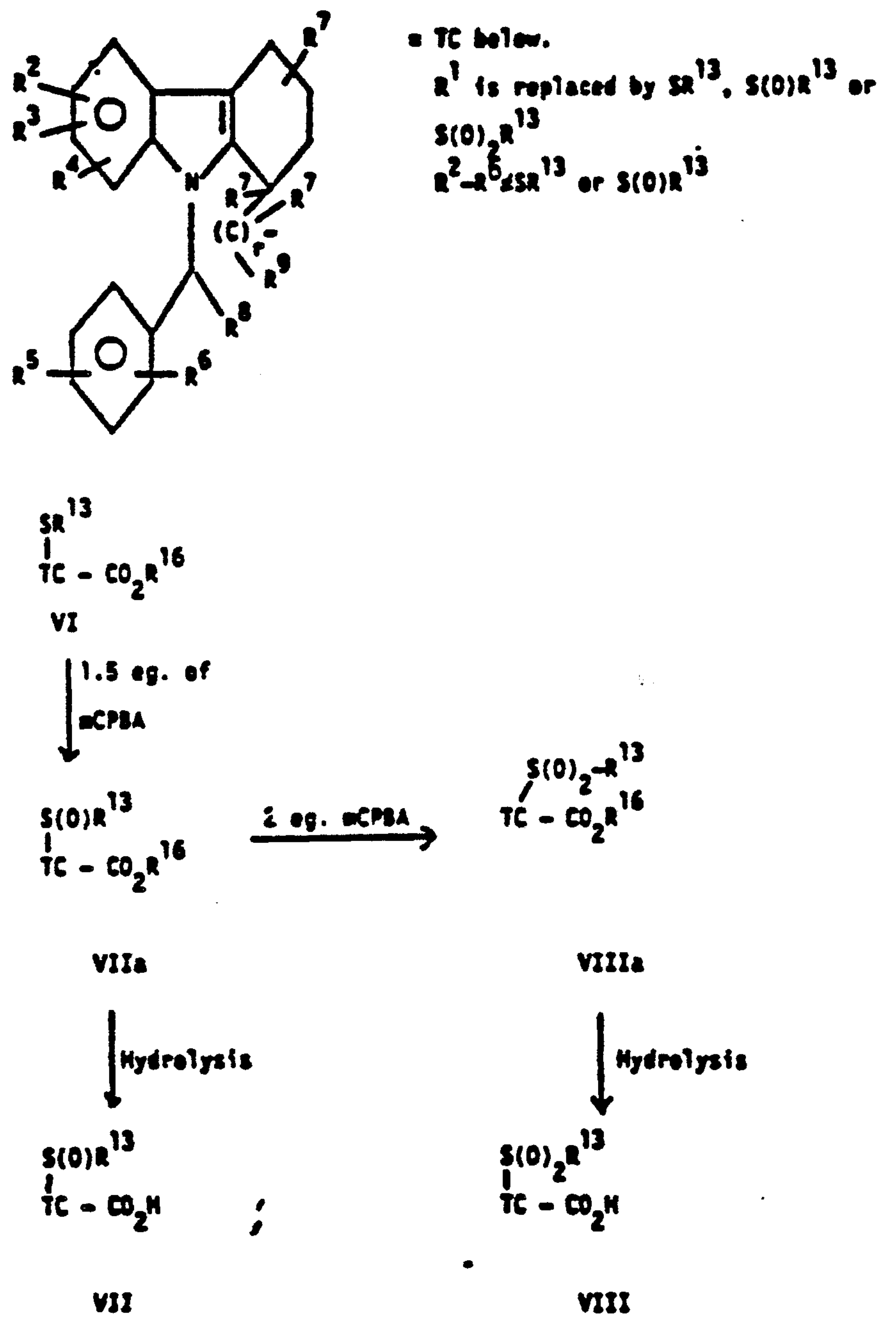


VI

VIIa

VIIIa

VII

VIII

In Scheme IV is illustrated a method of preparing derivatives of Formula I in which one of the substituents among $R^1$-$R^4$ is a sulfoxide or a sulfone. It will be obvious to one skilled in the art that a sulfoxide or sulfone derivative of $R^5$ or $R^6$ could be prepared in the same way.

Ester VI (a representative of III a, Scheme I) is prepared according to Scheme I or Scheme III followed by esterification of acid I. Treatment of VI with a limited amount of an oxidizing agent such as m-chloroperbenzoic acid yields the sulfoxide ester VIIa, which upon hydrolysis yields sulfoxide acid VII. Further treatment of VIIa with the oxidizing agent, or treatment of VI with an excess (>2 eq.) of the oxidizing agent yields the sulfone ester VIIIa, hydrolysis of which yields the sulfone acid VIII. Both VII and VIII are

representatives of Formula I compounds.

Scheme V — Preparation of Further compounds of Formula I

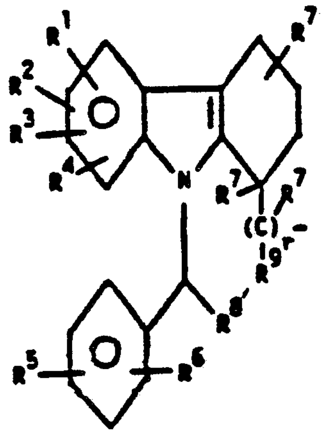

= THC below

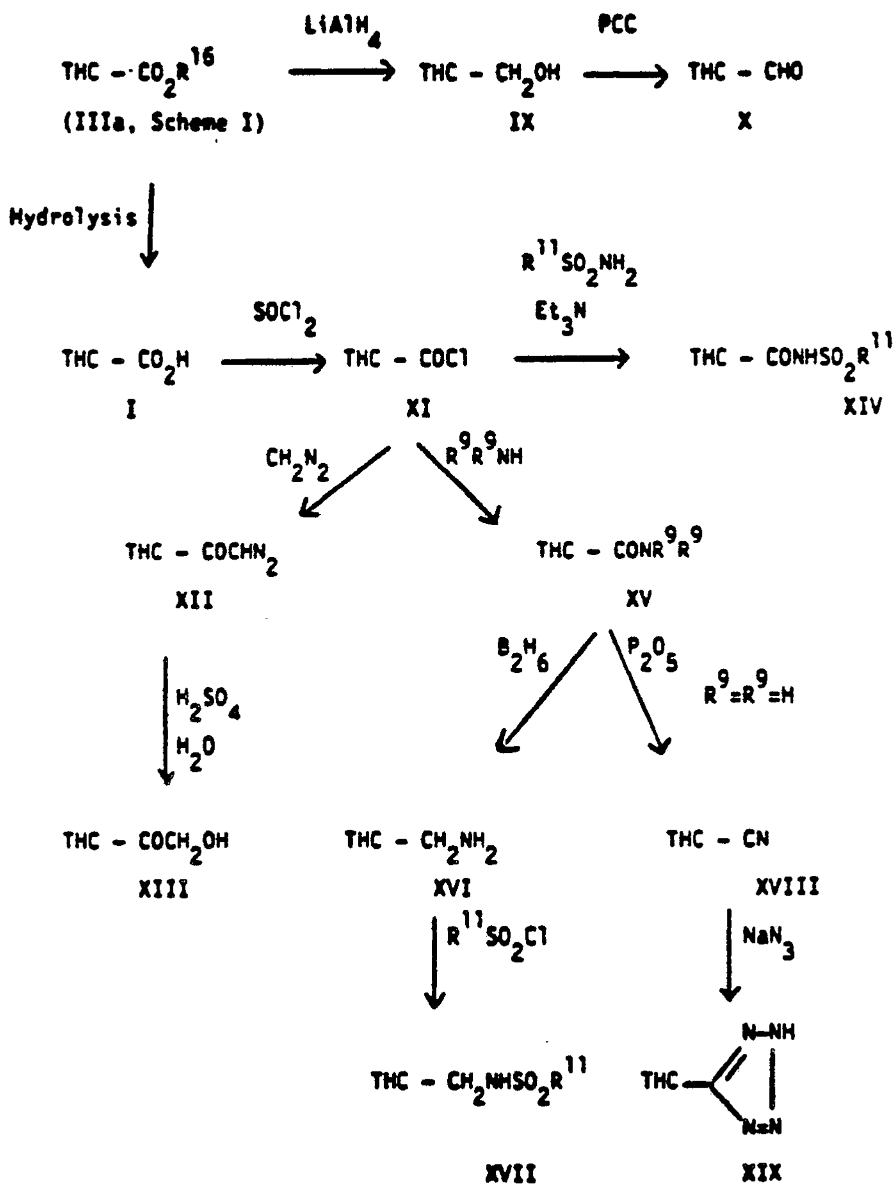

Other compounds of Formula I can be prepared as indicated in Scheme V. Thus the ester derivative IIIa can be reduced to the alcohol IX by lithium aluminum hydride or other suitable reducing agents. Alcohol IX can then be oxidized to aldehyde X by pyridinium chlorochromate or other suitable oxidizing agents. Carboxylic acids of Formula I can be converted to the acid chloride XI (the acid bromide or a mixed carbonate anhydride could also be used) which when reacted with diazomethane yields the diazoketone XII. Compound XII, upon reaction with aqueous acid, preferably a nonnucleophilic acid such as sulfuric acid or p-toluenesulfonic acid, is converted to the hydroxymethyl ketone XIII.

Acid chloride XI, upon reaction with a sulfonamide, $R^{11}SO_2NH_2$, in the presence of a weak base yields the acyl-sulfonamide XIV. Reaction of XI with an amine, $R^9R^9NH$, yields amide XV. Amide XV can be sequentially reduced, to amine XVI, with diborane or lithium aluminum hydride, and sulfonylated with $R^{11}SO_2Cl$ to produce sulfonamide XVII. Amide XV (when both $R^9$ substituents are hydrogen) can be dehydrated by standard reagents to nitrile XVIII, which is converted to tetrazole XIX by reaction with sodium azide, tri-n-butyltin azide or other suitable methods. Compounds IX, X, XIII, XIV, XV, XVII, XVIII and XIX are representatives of Formula I compounds.

Scheme VI Preparation of Hydrazine Deniratives IV

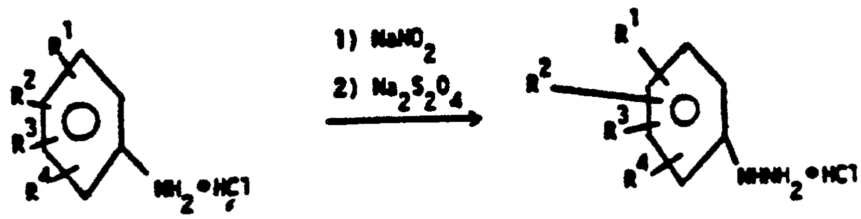


IV

With regard to Scheme VI, the preparation of hydrazine starting materials is illustrated by preparation of 4-methylthiophenyl hydrazine hydrochloride. 4-Methylthioaniline (13.9 g) was added dropwise to cold HCl (6N) (50 mL) and stirred for 5 min in an ice bath. A solution of $NaNO_2$ in water (7.25 g, 15 mL) was then added dropwise and stirred for 15 min. The cold diazonium salt was then cannulated into a stirred cold solution of $Na_2S_2O_4$ in water (50 g, 250 mL). After 20 min, ether (200 mL) was added and the reaction mixture basified with NaOH(10N). The ether layer was decanted, washed with brine, dried over $Na_2SO_4$ and HCl gas was passed through the ether solution to form the hydrochloride salt which precipitated out. After filtration, there was obtained 7.0 g of pure final product. Other hydrazines, similarly prepared, are also shown in Table 4, below.

TABLE 3a

HYDRAZINES

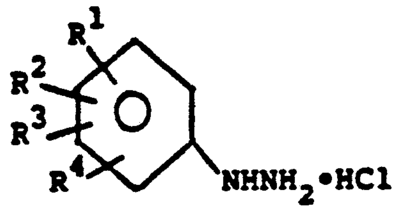

IV

| No. | $R^1$ | $R^2$ | Compound Name |
|---|---|---|---|
| 1 | 4-SMe | H | 4-methylthiophenyl hydrazine hydrochloride |

TABLE 3a (Cont'd)

| | | | |
|---|---|---|---|
| 2 | 2-CH(Me)$_2$ | H | 2-isopropylphenyl hydrazine hydrochloride |
| 3 | 2-SMe | H | 2-methylthiophenyl hydrazine hydrochloride |
| 4 | 2-Me | 4-Me | 2,4-dimethylphenyl hydrazine hydrochloride |
| 5 | 2-Me | 4-OMe | 4-methoxy-2-methylphenyl hydrazine hydrochloride |

$R^3$=$R^4$=H

TABLE 3b

Benzyl Halides V

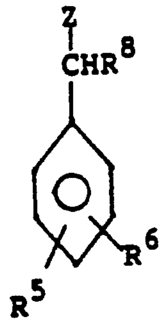

where $R^8$ is H

| Compound No. | Z | $R^5$ | $R^6$ | Compound Name |
|---|---|---|---|---|
| 1. | Cl | 4-Cl | H | 4-chlorobenzyl chloride (ALDRICH) |

| Compound No. | Z | $R_5$ | $R^6$ | Compound Name |
|---|---|---|---|---|
| 2. | Cl | 4-OMe | H | 4-methoxybenzyl chloride (ALDRICH) |
| 3. | Cl | 2-Cl | 4-Cl | 2,4-dichlorobenzyl chloride (ALDRICH) |
| 4. | Br | 2-Cl | H | 2-chlorobenzyl bromide (ALDRICH) |
| 5. | Br | 3-Cl | H | 3-chlorobenzyl bromide (ALDRICH) |
| 6. | Br | 4-F | H | 4-fluorobenzyl bromide (ALDRICH) |
| 7. | Br | 4-$CF_3$ | H | 4-trifluoromethyl- benzyl bromide (ALDRICH) |
| 8. | Cl | 4-$CO_2$Me | H | 4-carbomethoxybenzyl chloride (J.A.C.S. 1950, 72 5152) |
| 9. | Cl | 4-SMe | H | 4-methylthiobenzyl chloride (C.A.: 56: 4774 (1962)) |

| Compound No. | Z | $R^5$ | $R^6$ | Compound Name |
|---|---|---|---|---|
| 10. | Cl | 4-SOMe | H | 4-methylsulfinyl- benzyl chloride (C.A.: 84: 104277h (1976)) |
| 11. | Cl | 4-$SO_2$Me | H | 4-methylsulfonylbenzyl chloride (C.A.: 78: 1113259 (1973)) |
| 12. | Br | 4-$NO_2$ | H | 4-nitrobenzyl bromide (ALDRICH) |
| 13. | Cl | 4-$CONMe_2$ | H | 4-dimethylcarbox-amidobenzyl chloride |
| 14. | Cl | 4-SO2$NMe_2$ | H | 4-dimethylsulfon-amidobenzyl chloride (ALDRICH) |
| 15. | Cl | 4-$CO_2$H | H | 4-carboxybenzyl chloride (ALDRICH) |
| 16. | Cl | 4-COMe | H | 4-acetylbenzyl chloride (C.A.: 93: 2399945 (1930)) |

In those instances when asymmetric centers are present, more than one stereoisomer is possible, and all possible isomeric forms are deemed to be included within the planar structural representations shown. Optically active (R) and (S) isomers may be resolved using conventional techniques known to the skilled artisan.

The effective daily dosage level for compounds of Formula I in mammals, especially humans, will generally range from about 0.002 mg/kg to about 100 mg/kg, preferably from about 0.02 mg/kg to about 30 mg/kg. The dosage may be administered in single or divided individual doses.

Any suitable route of administration may be employed for providing a mammal, especially a human, with an effective dosage of a compound of Formula I. For example, oral, rectal, topical, parenteral, ocular, nasal, buccal, intravenous and the like may be employed. Dosage forms include tablets, troches, dispersions, suspensions, solutions, capsules, creams, ointments, aerosols and the like.

The compounds of Formula I can be part of a pharmaceutical composition as an active ingredient or a pharmaceutically acceptable salt thereof, and may also contain a pharmaceutically acceptable carrier and optionally other therapeutic ingredients. The term "pharmaceutically acceptable salts" refers to salts prepared from pharmaceutically acceptable non-toxic bases including inorganic bases and organic bases. The compositions include compositions suitable for oral, or parenteral (including subcutaneous, intramuscular and intravenous) administration. They may be conveniently presented in unit dosage form and

prepared by any of the methods well-known in the art of pharmacy.

In addition to the common dosage forms set out above, the compounds of Formula I may also be administered by controlled release means and/or delivery devices such as those described in U.S. Patent Nos. 3,845,770; 3,916,899; 3,536,809; 3,598,123; 3,630,200 and 4,008,719, the disclosure of which is hereby incorporated herein by reference.

Pharmaceutical compositions of the compounds of Formula I suitable for oral administration can be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Desirably, each tablet contains from about 2.5 mg to about 500 mg of the active ingredient and each cachet or capsule contains from about 2.5 to about 500 mg of the active ingredient.

The following are examples of representative pharmaceutical dosage forms for the compounds of Formula I:

| Injectable Suspension (I.M.) | mg/ml |
|---|---|
| Compound of Formula I | 2.0 |
| Methylcellulose | 5.0 |
| Tween 80 | 0.5 |
| Benzyl alcohol | 9.0 |
| Benzalkonium chloride | 1.0 |
| Water for injection to a total volume of 1 ml | |
| **Tablet** | **mg/tablet** |
| Compound of Formula I | 25.0 |
| Microcrystalline Cellulose | 415.0 |
| Providone | 14.0 |
| Pregelatinized Starch | 43.5 |
| Magnesium Stearate | 2.5 |
| | 500 |
| **Capsule** | **mg/capsule** |
| Compound of Formula I | 25.0 |
| Lactose Powder | 573.5 |
| Magnesium Stearate | 1.5 |
| | 600 |

The following examples illustrate the preparation of the compounds of Formula I without, however, limiting the same thereto.

All temperatures are in degrees Celsius.

## Reference Compounds

6-fluoro-1,2,3,4-tetrahydrocarbazol-1-yl-acetic acid

Following the procedure of Example 1, but using 1-(4-fluorophenyl) hydrazine hydrochloride and ethyl-2-cyclohexanone acetate as starting materials, the title compound was prepared.

M.P. 124 -126 °C

Reference Compounds

9-[1-(2-p-chlorophenyl)ethyl]-6-fluoro-1,2,3,4-tetrahydrocarbazol-1-yl-acetic acid

Following the procedure of Example 2, but using 1-[2-(4 chlorophenyl)ethyl]-1-(4-fluorophenyl) hydrazine hydrochloride and ethyl 2-cyclohexanone acetate as starting materials, the title compound was prepared.

| Empirical Formula: $C_{22}H_{21}NFClO_2$ | | | |
|---|---|---|---|
| | C | H | N |
| Calculated | 68.48 | 5.49 | 3.63 |
| Found | 68.15 | 5.70 | 3.65 |

Reference Compounds

6-fluoro-9-isopropyl-1,2,3,4 tetrahydrocarbazol-1-yl-acetic acid

Following the procedure of Example 1, but using 1-(2-propyl)-1-(4-fluorophenyl)hydrazine hydrochloride and ethyl 2-cyclohexanone acetate as starting materials, the title compound was prepared.

Empirical Formula: $C_{17}H_{20}NFO_2$

M.P. 144-144.5 °C

Example 1

9-p-chlorobenzyl-6-fluoro-1,2,3,4-tetrahydrocarbazol-1-yl-acetic acid

Step I

To 3.50 g of 1-(4-chlorobenzyl)-1-(4-fluorophenyl)hydrazine hydrochloride in 70 cc of isopropanol was added 2.23 g of ethyl 2-cyclohexanone acetate. The reaction was refluxed under nitrogen for 16 hours. The resulting reaction mixture was then evaporated to dryness and the residue suspended in ether. The solid material was then filtered. The ether filtrate was washed with water, dried and evaporated. The resulting syrup was chromatographed on silica gel to give 2.8 g (42%).

Step II

To 1.59 g of ethyl ester from step I in 10 cc of methanol was added 10 cc of water and 420 mg of potassium hydroxide. The resulting solution was refluxed for 4 hours. Upon cooling the reaction mixture was then acidified with HCl (1N). The resulting precipitate was filtered and washed with water. Analytically pure material was prepared by trituration the solid with a mixture of hexane/ethyl acetate (9:1) followed by filtration and drying on a high vacuum pump to give 1.24 g of the title compound (89%).

| Analysis calculated for $C_{21}H_{19}NClFO_2$ | | | | | |
|---|---|---|---|---|---|
| | C | H | N | Cl | F |
| Calculated | 67.83 | 5.15 | 3.77 | 9.53 | 5.11 |
| Found | 67.88 | 5.47 | 3.63 | 9.52 | 5.12 |

Example 2

3-[9-p-chlorobenzyl-6-fluoro-1,2,3,4-tetrahydrocarbazol-1-yl]-propanoic acid

Following the procedure of Example 1, but using 1-(4-chlorobenzyl)-1-(4-fluorophenyl)hydrazine hydrochloride and methyl-2-cyclohexanone propionate as starting materials, the title compound was prepared.

| Empirical formula: $C_{22}H_{21}ClFNO_2$ | | | |
|---|---|---|---|
| | C | H | N |
| Calculated | 68.48 | 5.49 | 3.63 |
| Found | 68.26 | 5.57 | 3.60 |

Example 3

3-[9-p-chlorobenzyl-6-methoxy-1,2,3,4-tetrahydrocarbazol-1-yl]-propanoic acid

Following the procedure of Example 1, but using 1-(4-chlorobenzyl)-1-(4-methoxyphenyl)hydrazine hydrochloride and methyl-2-cyclohexanone propionate as starting materials, the title compound was prepared.

| Empirical formula: $C_{22}H_{22}ClNO_3$ | | | | |
|---|---|---|---|---|
| | C | H | N | Cl |
| Calculated | 69.43 | 6.08 | 3.52 | 8.91 |
| Found | 69.24 | 5.98 | 3.60 | 8.85 |

Example 4

9-p-chlorobenzyl-6-methoxy-1,2,3,4-tetrahydrocarbazol-1-yl-acetic acid

Following the procedure of Example 1, but using 1-(4-chlorobenzyl)-1-(4-methoxyphenyl)hydrazine hydrochloride and ethyl-2-cyclohexanone acetate as starting materials, the title compound was prepared.
M.P. 152-153 °C.

## Example 5

2-[9-p-chlorobenzyl-6-fluoro-1,2,3,4-tetrahydrocarbazol-1-yl]ethanol

1.10 g of 9-p-chlorobenzyl-6-fluoro-1,2,3,4-tetrahydrocarbazo-1-yl-acetic acid ethyl ester was dissolved in 500 ml dry tetrahydrofuran and the reaction was cooled at 0 °C and one equivalent of lithium aluminum hydride (LAH) was added portion wise. The reaction was allowed to warm up to room temperature and stirred for 16 hrs. The reaction mixture was quenched with $NH_4Cl$ (aq.). Ethyl acetate was added (100 ml) and the organic phase separated, washed with water and brine, dried and evaporated. The product was isolated by column chromatography.
M.P. 98.0 - 98.5 °C.

## Example 6

3-[9-p-chlorobenzyl-6-methoxy-1,2,3,4-tetrahydrocarbazol-1-yl]-propanol

Following the procedure of Example 5, but using 3-[9-p-chlorobenzyl-6-methoxy-1,2,3,4-tetrahydrocarbazo-1-yl]-propanoic acid methyl ester from Example 3 as starting material, the title compound was prepared.

| Empirical formula: $C_{23}H_{26}ClNO_2$ | | | |
|---|---|---|---|
| | C | H | N |
| Calculated | 71.95 | 6.83 | 3.65 |
| Found | 71.86 | 6.65 | 3.81 |

## Example 7

(-)9-p-chlorobenzyl-6-fluoro-1,2,3,4 tetrahydrocarbazol-1-yl-acetic acid

Step I

10.0 g of 9-p-chlorobenzyl-6-fluoro-1,2,3,4-tetrahydrocarbazo-1-yl-acetic acid from Example 1 was dissolved in a mixture of hot (refluxing) acetonitrile (150 cc), and ethanol (25cc) and 4.4 g of d(+) ephedrine was added. The reflux was continued for 15 min. and the hot solution was filtered and allowed to cool to room temperature. Crystals separated from the solution and were separated by filtration. After three recrystallizations from acetonitrile 3.9 g of the pure salt was obtained.

Step II

3.9 g of pure salt from step I was dissolved in 200 cc of methanol and acidified using 1 N hydrochloric acid. Water was added and the crystals were separated by filtration and dryed under vacuum. Upon trituration with hexane ethyl acetate mixture (9:1) the title compound was prepared.

aD = -42.5 (methanol) M.P. 151 - 151.5° C.

## Example 8

(+)9-p-chlorobenzyl-6-fluoro-1,2,3,4-tetrahydrocarbazol-1-yl-acetic acid

Following the method of Example 7, but using 1(-) ephedrine and 9-p-chlorobenzyl-6-fluoro-1,2,3,4-tetrahydrocarbazo-1-yl acetic acid as starting material, the title compound was prepared.

aD = +43.0 (methanol) M.P. 150 - 150.5° C.

## Example 9

9-benzyl-6-fluoro-1,2,3,4-tetrahydrocarbazol-1-yl-acetic acid

Following the procedure of Example 1, but using 1-(benzyl)-1- (4-fluorophenyl)hydrazine hydrochloride and ethyl 2-cyclohexanone acetate as starting materials, the title compound was prepared.

| Analysis calculated for $C_{21}H_{20}NFO_2$ | | | |
|---|---|---|---|
| | C | H | N |
| Calculated | 74.76 | 5.98 | 4.15 |
| Found | 74.95 | 6.07 | 3.90 |

## Example 10

9-p-methoxybenzyl-6-fluoro-1,2,3,4-tetrahydrocarbazol-1-yl-acetic acid

Following the procedure of Example 1, but using 1-(4-methoxybenzyl)-1-(4-fluorophenyl)hydrazine hydrochloride and ethyl 2-cyclohexanone acetate as stating materials, the title compound was prepared.

| Analysis calculated for $C_{22}H_{22}NFO_3$ | | | |
|---|---|---|---|
| | C | H | N |
| Calculated | 71.92 | 6.04 | 3.91 |
| Found | 71.70 | 6.22 | 4.05 |

## Example 11

9-(3,4-dichloro)benzyl-6-fluoro-1,2,3,4-tetrahydrocarbazol-1-yl-acetic acid

Following the procedure of Example 1, but using 1-(3,4-dichlorobenzyl)-1-(4-fluorophenyl) hydrazine hydrochloride and ethyl 2-cyclohexanone acetate as starting materials, the title compound was prepared.

| Analysis calculated for $C_{21}H_{18}NCl_2FO_2$ | | | |
|---|---|---|---|
| | C | H | N |
| Calculated | 62.08 | 4.47 | 3.45 |
| Found | 61.96 | 4.71 | 3.67 |

Example 12

9-[1-(1 phenyl)ethyl]-6-fluoro-1,2,3,4 tetrahydrocarbazol-1-yl-acetic acid

Following the procedure of Example 1, but using 1-[1-(1-phenyl)ethyl]-1-(4-fluorophenyl)hydrazine hydrochloride and ethyl 2-cyclohexanone acetate as starting materials, the title compound was prepared.

| | C | H | N | F |
|---|---|---|---|---|
| Calculated | 75.19 | 6.31 | 3.99 | 5.41 |
| Found | 75.18 | 6.05 | 4.11 | 5.51 |

Example 13

9-p-chlorobenzyl-1,2,3,4-tetrahydrocarbazol-1-yl-acetic acid

Following the procedure of Example 1, but using 1-(4-chlorobenzyl)-1-(phenyl)hydrazine hydrochloride and ethyl 2-cyclohexanone acetate as starting materials, the title compound was prepared.

| Empirical Formula: $C_{21}H_{20}NClO_2$ | | | | |
|---|---|---|---|---|
| | C | H | N | Cl |
| Calculated | 71.28 | 5.70 | 3.96 | 10.02 |
| Found | 70.7n | 5.90 | 3.82 | 10.23 |

Example 14

9-p-chlorobenzyl-6-chloro-1,2,3,4-tetrahydrocarbazol-1-yl-acetic acid

Following the procedure of Example 1, but using 1-(4-chlorobenzyl)-1-(4-chlorophenyl)hydrazine hydrochloride and ethyl-2-cyclohexanone acetate as starting materials, the title compound was prepared.

| Empirical Formula: $C_{21}H_{19}NCl_2O_2$ | | | | |
|---|---|---|---|---|
| | C | H | N | Cl |
| Calculated<br>Found | 69.96<br>65.20 | 4.93<br>5.16 | 3.61<br>3.38 | 18.26<br>18.04 |

## Example 15

9-p-chlorobenzyl-8-methyl-1,2,3,4-tetrahydrocarbazol-1-yl-acetic acid

Following the procedure of Example 1, but using 1-(4-chlorobenzyl)-1-(2-methylphenyl)hydrazine hydrochloride and ethyl-2-cyclohexanone acetate as starting materials, the title compound was prepared.

| Empirical Formula: $C_{22}H_{22}NClO_2$ | | | | |
|---|---|---|---|---|
| | C | H | N | Cl |
| Calculated<br>Found | 71.83<br>72.19 | 6.03<br>6.23 | 3.81<br>4.12 | 9.64<br>9.84 |

## Example 16

6-bromo-9-p-chlorobenzyl-1,2,3,4-tetrahydrocarbazol-1-yl-acetic acid

Following the procedure of Example 1, but using 1-(4-chlorobenzyl)-1-(4-bromophenyl)hydrazine hydrochloride and ethyl 2-cyclohexanone acetate as starting materials, the title compound was prepared.

| Empirical formula: $C_{21}H_{19}BrClNO_2$ | | | | | |
|---|---|---|---|---|---|
| | C | H | N | Br | Cl |
| Calculated .<br>Found | 58.29<br>58.49 | 4.43<br>4.60 | 3.24<br>3.44 | 18.46<br>18.50 | 8.19<br>8.27 |

## Example 17

9-p-chlorobenzyl-6-methyl-1,2,3,4-tetrahydrocarbazol-1-yl-acetic acid

Following the procedure of Example 1, but using 1-(4-chlorobenzyl)-1-(4-methylphenyl)hydrazine hydrochloride and ethyl 2-cyclohexanone acetate as starting materials, the title compound was prepared.

| Empirical formula: $C_{22}H_{22}ClNO_2$ | | | | |
|---|---|---|---|---|
| | C | H | N | Cl |
| Calculated<br>Found | 71.83<br>71.72 | 6.03<br>6.14 | 3.81<br>3.77 | 9.64<br>9.88 |

Example 18

2-[9-p-chlorobenzyl-6-fluoro-1,2,3,4-tetrahydrocarbazol-1-yl]propanoic acid.

Following the procedure of Example 1, but using 1-(4 chlorobenzyl)-1-(4-fluorophenyl) hydrazine hydrochloride and methyl 2-(2-cyclohexanone) propionate as starting materials, the title compound was prepared.

M.P. 203-205° C

Example 19

9-p-chlorobenzyl-8 fluoro-1,2,3,4-tetrahydrocarbazol-1-yl-acetic acid

Following the procedure of Example 1, but using 1-(4-chlorobenzyl)-1-(2-fluorophenyl) hydrozine hydrochloride and ethyl 2-cyclohexanone acetate as starting materials, the title compound was prepared.

M.P. 228-229° C.

Example 20

3-[( a and β)-t-butyl-6-fluoro-9-(p-chlorobenzyl)-1,2,3,4-tetrahydrocarbazol-1-yl]acetic acid.

Following the procedure of Example 1 but using 4-t-butyl-2-carbomethoxymethyl cyclohexanone and 1-(4-chlorobenzyl)-4-fluorophenylhydrazine hydrochloride as the starting materials, the title compound was prepared.

M.P. 210-211° C.

Example 21

9-p-chlorobenzyl-5-fluoro-1,2,3,4-tetrahydrocarbazol-1-yl acetic acid and 9-p-chlorobenzyl-7-fluoro-1,2,3,4-tetrahydocarbazol-1-yl-acetic acid (mixture).

Following the procedure of Example 1, but using 1-(4 chlorobenzyl)-1-(3-fluorophenyl)hydrazine hydrocloride and ethyl 2-cyclohexanone acetate as starting materials, the title compounds were prepared.

Empirical Formula: $C_{21}H_{19}NClFO_2$

M. P. 211-219° C

Example 22

9-p-chlorobenzyl-5,7-dichloro-1,2,3,4-tetrahydrocarbazol-1-yl-acetic acid.

Following the procedure of Example 1, but using 1-(4-chlorobenzyl)-1-(3-5 dichlorophenyl)hydrozine hydrochloride and ethyl 2-cyclohexanone acetate as starting materials, the title compound was prepared.

Empirical formula: $C_{21}H_{18}NCl_3O_2$

M.P. 204-206 °C

Example 23

9-p-chlorobenzyl-6,8-dichloro-1,2,3,4-tetrahydrocarbazol-1-yl-acetic acid.

Following the procedure of Example 1, but using 1-(4-chlorobenzyl)-1-(2-4-dichlorophenyl)hydrozine hydrochloride and ethyl 2-cyclohexanone acetate as starting materials, the title compound was prepared.

Empirical formula: $C_{21}H_{18}NCl_3O_2$

M.P. 203-204 °C.

Example 24

9-p-Chlorobenzyl-6-isopropyl-1,2,3,4-tetrahydrocarbazol-1-yl-acetic acid

Following the procedure of Example 1, but using 1(4-chlorobenzyl)-1-(4-isopropylphenyl)hydrazine hydrochloride and ethyl 2-cyclohexanone acetate as starting material, the title compound was prepared.

| Empirical Formula: $C_{24}H_{26}ClNO_2$ | | | | |
|---|---|---|---|---|
| | C | H | N | Cl |
| Calc: | 72.81 | 6.62 | 3.54 | 8.95 |
| Found: | 72.83 | 7.09 | 3.61 | 9.21 |

Example 25

9-p-Chlorobenzyl-6-tert-butyl-1,2,3,4-tetrahydrocarbazol-1-yl-acetic acid

Following the procedure of Example 1, but using 1-(4-chlorobenzyl)-1-(4-tert-butylphenyl) hydrazine hydrochloride and ethyl2-cyclohexanone acetate as starting materials, the title compound was prepared.

| Empirical Formula: $C_{25}H_{28}ClNO_2$ | | | | |
|---|---|---|---|---|
| | C | H | N | Cl |
| Calc. | 73.24 | 6.88 | 3.42 | 8.65 |
| Found: | 73.21 | 7.32 | 3.10 | 8.26 |

## Example 26

9-p-Chlorobenzyl-6-trifluoromethyl-1,2,3,4-tetrahydrocarbazol-1-yl-acetic acid

Following the procedure of Example 1, but using 1-(4-chlorobenzyl)-1-(4-trifluoromethylphenyl) hydrazine hydrochloride and ethyl 2-cyclohexanone acetate as starting materials, the title compound was prepared. m.p. 167-168° C.

## Example 27

9-p-Chlorobenzyl-6-methylthio-1,2,3,4-tetrahydro carbazol-1-yl-acetic acid

Following the procedure of Example 1, but using 1-(4-chlorobenzyl)-1-(4-methylthiophenyl) hydrazine hydrochloride and ethyl 2-cyclohexanone acetate as starting materials, the title compound was prepared.

| Empirical Formula: $C_{22}H_{22}ClNO_2S$ | | | | | |
|---|---|---|---|---|---|
| | C | H | N | S | Cl |
| Calc: | 66.07 | 5.54 | 3.50 | 8.02 | 8.86 |
| Found: | 66.27 | 5.83 | 3.38 | 8.00 | 8.70 |

## Example 28

9-p-Chlorobenzyl-6-methylsulfinyl-1,2,3,4-tetrahydrocarbazol-1-yl-acetic acid

### Step I

To 498 mg of ethyl 9-p-chlorobenzyl-6-methylthio-1,2,3,4-tetrahydrocarbazol-1-yl-acetate from Example 27, Step I, in 10 cc of methylene chloride was added 300 mg of m-chloro perbenzoic acid. The resulting mixture was stirred for 1.5 hours at room temperature. The reaction mixture was diluted with ether and washed consecutively with a solution of sodium bicarbonate, water and brine. The crude product obtained after evaporation of the organic layer was purified on silica gel by flash chromatography eluting with 20% hexane/ethyl acetate and yielded 420 mg (82%) of the pure sulfoxide derivative.

### Step II

Following the procedure of Example 1, Step II, but using the ethyl ester from Step I, there was obtained the title compound. m.p. 105-107° C.

| Empirical Formula: $C_{22}H_{22}ClNO_3S$ | | | | | |
|---|---|---|---|---|---|
| | C | H | N | S | Cl |
| Calc: | 63.53 | 5.33 | 3.37 | 7.71 | 8.52 |
| Found: | 63.31 | 5.03 | 2.94 | 7.69 | 8.48 |

Example 29

9-p-Chlorobenzyl-6-methylsulfonyl-1,2,3,4-tetrahydrocarbazol-1-yl-acetic acid

Step I

To 439 mg of ethyl 9-p-chlorobenzyl-6-methylsulfinyl-1,2,3,4-tetrahydrocarbazol-1-yl-acetate from Example 28, Step I, in 10 cc of methylene chloride was added 353 mg of m-chloro perbenzoic acid. The resulting mixture was stirred for 18 hours at room temperature. The reaction mixture was diluted with ether and washed consecutively with a solution of sodium bicarbonate, water and brine. The crude product obtained after evaporation of the organic layer was purified on silica gel by flash chromatography eluting with 30% hexane/ethyl acetate and yielded 200 mg (42%) of the pure sulfone derivative.

Step II

Following the procedure of Example 1, Step II, but using the ethyl ester from Step I, there was obtained the title compound. m.p. 101-102° C.

Example 30

9-p-Chlorobenzyl-8-isopropyl-1,2,3,4-tetrahydrocarbazol-1-yl-acetic acid

Following the procedure of Example 1, but using 1-(4-chlorobenzyl)-1-(2 isopropylphenyl) hydrazine hydrochloride and ethyl 2 cyclohexanone acetate as starting materials. the title compound was prepared.

| Empirical Formula: $C_{24}H_{26}ClNO_2$ | | |
|---|---|---|
| | C | H |
| Calc: | 72.81 | 6.62 |
| Found: | 72.59 | 6.90 |

Example 31

9-p-Chlorobenzyl-8-methylthio-1,2,3,4-tetrahydrocarbazol-1-yl-acetic acid

Following the procedure of Example 34, but using 1-(2-methylthiophenyl) hydrazine hydrochloride and ethyl 2-cyclohexanone acetate as starting materials, the title compound was prepared. m.p. 141-142° C.

Example 32

9-p-Chlorobenzyl-8-methylsulfinyl-1,2,3,4-tetrahydrocarbazol-1-yl-acetic acid

Following the procedure of Example 28, but using the ethyl ester from Example 31, Step II, there was obtained the title compound. m.p. 119-120.5° C.

## Example 33

9-p-Chlorobenzyl-6-fluoro-3-methyl-1,2,3,4-tetrahydrocarbazol-1-yl-acetic acid

Following the procedure of Example 1, but using 1-(4-chlorobenzyl)-1-(4-fluorophenyl) hydrazine hydrochloride and ethyl 4-methyl-2-cyclohexanone acetate as starting materials, the title compound was prepared. m.p. 205-206° C.

| Empirical Formula: $C_{22}H_{21}FClNO_2$ | | | | |
|---|---|---|---|---|
| | C | H | N | Cl |
| Calc: | 68.48 | 5.49 | 3.63 | 9.19 |
| Found: | 68.80 | 5.50 | 3.30 | 9.47 |

## Example 34

9-p-Chlorobenzyl-6,8-difluoro-1,2,3,4-tetrahydrocarbazol-1-yl-acetic acid

Step I

To 114 g of 1-(2,4-difluorophenyl) hydrazine hydrochloride in 350 cc of 2-propanol containing 40 cc of acetyl chloride was added 138 g of ethyl 2-cyclohexanone acetate. The reaction was refluxed under nitrogen for 2 days After cooling, 200 cc of ether was added and the precipitate filtered. The filtrate was evaporated to dryness. The resulting residue was dissolved in a (1:1) mixture of ether/ethyl acetate and consecutively washed with water, sodium bicarbonate solution and brine. The organic layer was dried over sodium sulfate and evaporated to dryness. The crude product was passed through a silica gel bed eluting with 5% ethyl acetate/hexane to yield 84 g of a 1:2 mixture of ethyl and isopropyl esters.

Step II

84 g of esters from Step I was dissolved in 250 cc of methanol and 400 cc of sodium hydroxide (1N) was added and refluxed 4 hours. After cooling, the reaction mixture was washed with a (1:1) mixture of ether/hexane and the aqueous layer was acidified with HCl (1N). The resulting precipate was filtered, washed with water and air dried to afford 50 g of 6,8-difluoro-1,2,3,4-tetrahydrocarbazol-1-yl-acetic acid.

Step III

A solution of 11:1 g of acid from Step II in 100 cc of THF was added portionwise 10.3 g of potassium tert-butoxide. The resulting mixture was stirred for 45 min. at room temperature and 10.3 g p-chlorobenzyl bromide was added portionwise. The reaction mixture was stirred 18 hours at room temperature. The resulting mixture was diluted with 100 cc of water and washed with hexane. The aqueous layer was acidified with HCl (1N) and the resulting precipitate filtered washed with water and air-dried to afford 9.4 g of the title compound. m.p. 168.5-170 °C.

Example 35

9-p-Chlorobenzyl-6,8-dimethyl-1,2,3,4-tetrahydrocarbazol-1-yl-acetic acid

Following the procedure of Example 34, but using 1-(2,4-dimethylphenyl) hydrazine hydrochloride and ethyl 2-cyclohexanone acetate in Step I as starting materials, the title compound was prepared. m.p. 187-188 °C.

Example 36

9-p-Chlorobenzyl-6-methoxy-8-methyl-1,2,3,4-tetrahydrocarbazol-1-yl-acetic acid

Following the procedure of Example 34, but using 1-(4-methoxy-2 methylphenyl) hydrazine hydrochloride and ethyl 2 cyclohexanone acetate as starting materials, the little compound was prepared. m.p. 188-188.5 °C.

Example 37

(-)-9 p-Chlorobenzyl-6,8-difluoro-1,2,3,4-tetrahydrocarbazol-1-yl-acetic acid

Following the method of Example 7, but using d(+)ephedrine and 9-p-chlorobenzyl-6,8-difluoro-1,2,3,4-tetrahydrocarbazol-1-yl-acetic acid from Example 34 as starting materials, the title compound was prepared. From Example 34 $[a]_D$ = -64.3° (methanol) m.p. 130-131 °C.

Example 38

(+)-9-p-Chlorobenzyl-6,8-difluoro-1,2,3,4 tetrahydrocarbazol-1-yl-acetic acid

Following the method of Example 37, but using 1(-)ephedrine, the title compound was prepared. $[a]_D$ = +61.5° (methanol) m.p. 129.5-130 °C.

Example 39

(-)-9-p-Chlorobenzyl-8-methyl-1,2,3,4-tetrahydrocarbazol-1-yl-acetic acid

Following the method of Example 7, but using d(+)ephedrine and 9-p-chlorobenzyl-8-methyl-1,2,3,4-tetrahydrocarbazol-1-yl-acetic acid from Example 15 as starting materials, the title compound was prepared. [ a ]$_D$ = -51.6° (methanol) m.p. 196-198° C.

Example 40

(+)-9-p-Chlorobenzyl-8-methyl-1,2,3,4-tetrahydrocarbazol-1-yl-acetic acid

Following the method of Example 39, but using 1(-)ephedrine, the title compound was prepared. [ a ]$_D$ = +45.9° (methanol) m.p. 195-197° C.

Example 41

(-)-9-p-Chlorobenzyl-8-fluoro-1,2,3,4-tetrahydrocarbazol-1-yl-acetic acid

Following the method of Example 7, but using d(+)ephedrine and 9-p-chlorobenzyl-8-fluoro-1,2,3,4-tetrahydrocarbazol-1-yl-acetic acid from Example 19 as starting materials, the title compound was prepared. [ a ]$_D$ = -62.1° (methanol) m.p. 74-75° C.

Example 42

(+)-9-p-Chlorobenzyl-8-fluoro-1,2,3,4 tetrahydrocarbazol-1-yl-acetic acid

Following the method of Example 41, but using 1(-)ephedrine, the title compound was prepared. [ a ]$_D$ = +65.2° (methanol) m.p. 94-94.5° C.

Example 43

2-(9-p-Chlorobenzyl-6,8-difluoro-1,2,3,4-tetrahydrocarbazol-1-yl)ethanol

Following the procedure of Example 5, but using a mixture of 9-p-chlorobenzyl-6,8-difluoro- 1,2,3,4-carbazole--yl-acetic acid ethyl and isopropyl esters from Example 34 as starting materials, the title compound is obtained.

Example 44

(-) or (+) 2-(9-p-Chlorobenzyl-6,8-difluoro-1,2,3,4-tetrahydrocarbazol-1-yl)ethanol

Following the procedure of Example 5, but using (-) or (+) 9-p-chlorobenzyl-6,8-difluoro-1,2,3,4-tetrahydrocarbazol-1-yl-acetic acid from Example 37 or 38 as starting material, the title compounds are obtained.

Example 45

9-o-Chlorobenzyl-6,8-difluoro-1,2,3,4-tetrahydrocarbazol-1-yl-acetic acid

A solution of 200 mg of 6,8-difluoro-1,2,3,4-tetrahydrocarbazol-1-yl-acetic acid from Example 34 step II in 8 cc of DMF was added portionwise 40 mg of sodium hydride. The resulting mixture was stirred for 30 minutes at room temperature and 185 mg of o-chloro-benzyl bromide was then added. After stirring overnight at room temperature, the resulting mixture was diluted with water and washed with ether. The aqueous layer was acidified with (1N) HCl and extracted with ether. The ethereal layer was washed with brine, dried over $Na_2SO_4$ and evaporated to dryness. The crude product was purified on preparative plates (silica gel) eluting with $CHCl_3$:MeOH:$NH_4OH$ (8:4:1) to yield 98 mg of pure title product. m.p. 197-198 °C.

Example 46

9-(2,4-Dichlorobenzyl)-6,8-difluoro-1,2,3,4-tetrahydro-carbazol-1-yl-acetic acid

Following the procedure of Example 45, but substituting 2,4-dichlorobenzyl chloride for o-chlorobenzyl bromide as starting material, the title compound was obtained; m.p. 160-161 °C.

Example 47

9-p-Methylthiobenzyl-6,8-difluoro-1,2,3,4-tetrahydrocarbazol-1-yl-acetic acid

Following the procedure of Example 45, but substituting p-methylthiobenzyl chloride for o-chlorobenzyl bromide as starting material, the title compound was obtained. m.p. 145-146 °C.

Example 48

9-p-Methylsulfinylbenzyl-6,8-difluoro-1,2,3,4-tetrahydrocarbazol-1-yl-acetic acid

Following the procedure of Example 45, but substituting p-methylsulfinylbenzyl chloride for o-chlorobenzyl bromide as starting material, the title compound was obtained. m.p. 186-188 °C.

Example 49

9-p-methylsulfonylbenzyl-6,8-difluoro-1,2,3,4-tetrahydrocarbazol-1-yl-acetic acid

To a cold solution of (0 °C) of 4.0 g of 6,8-difluoro-1,2,3,4-tetrahydrocarbazol-1-yl-acetic acid in 75 cc of tetrahydrofuran was added dropwise 46.3 cc of a solution of KHMDS in toluene (0.684 M) and stirred for 10 minutes. To the resulting cold (0 °C) solution was added dropwise 3.7 g of a solution of p-methylsulfonylbenzyl chloride in 12 cc of tetrahydrofuran. The reaction mixture was then stirred at room temperature for 2

hours. The reaction mixture was diluted with water and washed with ether. The aqueous layer was acidified with (1N) HCl and extracted with ethyl acetate. The organic layer was washed with brine, dried over $Na_2SO_4$ and evaporated to dryness. The residue was triturated with a mixture of ethyl acetate:hexane (3:7) and filtered to yield 5.1 g of the title product. m.p. 217-219 °C.

## Example 50

(-)9-p-Methylsulfonylbenzyl-6,8-difluoro-1,2,3,4-tetrahydrocarbazol-1-yl acetic acid

Following the method of Example 7, but using 1(-) ephedrine and 9-p-methylsulfonylbenzyl-6,8-difluoro-1,2,3,4-tetrahydrocarbazol-1-yl-acetic acid from Example 49 as starting materials, the title compound was prepared $[a]_D$ = -56. °C (methanol).

## Example 51

(+)9-p-Methylsulfonylbenzyl-6,8-difluoro-1,2,3,4-tetrahydrocarbazol-1-yl-acetic acid

Following the method of Example 50, but using d(+) ephedrine, the title compound was prepared $[a]_D$ = +56.0° (methanol).

## Example 52

9-p-Trifluoromethylbenzyl-6,8-difluoro-1,2,3,4-tetrahydrocarbazol-1-yl-acetic acid

Following the procedure of Example 45, but substituting p-trifluoromethylbenzyl bromide for o-chlorobenzyl bromide as starting material, the title compound was obtained. m.p. 176-177 °C.

## Example 53

9-p-Fluorobenzyl-6,8-difluoro-1,2,3,4-tetrahydrocarbazol-1-yl-acetic acid

Following the procedure of Example 45, but substituting p-fluorobenzyl bromide for o-chlorobenzyl bromide as starting material, the title compound was obtained. m.p. 195-196 °C.

## Example 54

9-m-Chlorobenzyl-6,8-difluoro-1,2,3,4-tetrahydrocarbazol-1-yl-acetic acid

Following the procedure of Example 45, but substituting m-chlorobenzyl bromide for o-chlorobenzyl bromide as starting material, the title compound was obtained. m.p. 188-189 °C.

## Example 55

### 9-p-Carbomethoxybenzyl-6,8-difluoro-1,2,3,4-tetrahydrocarbazol-1-yl-acetic acid

Following the procedure of Example 45, but substituting p-carbomethoxybenzyl chloride for o-chlorobenzyl bromide as starting material, the title compound was obtained. m.p. 169-170 °C.

## Example 56

### 9-p-Dimethylcarboxamidobenzyl-6,8-difluoro-1,2,3,4-tetrahydrocarbazol-1-yl-acetic acid

Following the procedure of Example 45, but substituting p-dimethylcarboxamidobenzyl chloride for o-chlorobenzyl bromide as starting material, the title compound was obtained. m.p. 208-210 °C.

## Example 57

### 9-p-Acetylbenzyl-6,8-difluoro-1,2,3,4-tetrahydrocarbazol-1-yl-acetic acid

Following the procedure of Example 45, but substituting p-acetylbenzyl chloride for o-chlorobenzyl bromide as starting material, the title compound was obtained. m.p. 163-164 °C.

## Example 58

### 9-p-Dimethylaminosulfonylbenzyl-6,8-difluoro-1,2,3,4-tetrahydro carbazole-1-acetic acid

Following the procedure of Example 45, but substituting p-dimethylaminosulfonylbenzyl chloride for o-chloro benzyl bromide as starting material, the title compound was obtained m.p. 200-202 °C.

## Example 59

### 9-p-Acetamidobenzyl-6,8-difluoro-1,2,3,4 tetra-hydro carbazol-1-yl-acetic acid

#### Step I

Following the procedure of Example 45, but substituting p-nitrobenzyl bromide for o-chlorobenzyl chloride as starting material, the crude 9-p-nitrobenzyl-6,8-difluoro-1,2,3,4-tetrahydrocarbazol-1-yl-acetic acid was obtained.

Step II

The crude acid from step I was dissolved in ether and esterified with diazomethane. The reaction was monitored by TLC. The resulting solution was evaporated to dryness and the oily residue was chromatographed on flash silica gel column eluting with ethylacetate:hexane mixture (1:4) to afford 4.1 g (from 4.1 g of 6,8-difluoro-1,2,3,4-tetrahydrocarbazol-1-yl-acetic acid used in step I) of pure methyl 9-p-nitrobenzyl-6,8-difluoro-1,2,3,4-tetrahydrocarbazol-1-yl-acetate.

Step III

To a solution of 4.0 g of ester from step II in 40cc of ethyl acetate and 70 cc of ethanol was added 400 mg of Pd/c (10%) and the resulting mixture was hydrogenated on the parr for 90 minutes under 30 psi of hydrogen. The reaction mixture was filtered on celite and the filtrate was evaporated to dryness leaving 3.5 g of the aminoester derivative as a foam.

Step IV

To a solution of 595 mg of the aminoester derivative from step III and 0.325 cc of triethylamine in 10 cc of tetrahydrofuran was added dropwise 0.132 cc of acetyl chloride and the resulting mixture was stirred for 30 minutes at room temperature. The reaction mixture was diluted with water and ether. The ether layer was decanted, washed with brine, dried over $Na_2SO_4$ and evaporated to dryness. The residue was triturated with a mixture of ethylacetate:hexane (3:7) and filtered to afford 600 mg of the acetamidoester derivative.

Step V

A solution of 600 mg of the acetamidoester derivative from step IV in a 20 cc mixture of (2.5N) NaOH:ethanol (1:1) was stirred for 30 minutes at room temperature. The reaction mixture was acidified with (1N) HCl and the precipitate was filtered, washed with water and air dried to afford 500 mg of the title product m.p. 237°-239° C.

## Example 60

9-p-Methylsulfonamidobenzyl-6,8-difluoro-1,2,3,4-tetrahydrocarbazol-1-yl-acetic acid

Step I

Following the procedure of Example 59, step III and step IV, but substituting methanesulfonyl chloride for acetyl chloride as starting material, the title produce was obtained m.p. 196-198° C.

## Example 61

9-p-Methylureidobenzyl-6,8 difluoro-1,2,3,4-tetrahydrocarbazol-1-yl-acetic acid

To a solution of 538 mg of the aminoester derivative from Example 59 step III in 10 cc of tetrahydrofuran was added 0.3 cc of methylisocyanate and the resulting solution was stirred overnight. The reaction mixture was diluted with water and ether. The ether layer was decanted, washed with brine, dried over $Na_2SO_4$ and evaporated to dryness. The residue was triturated with ether and filtered to afford 532 mg of the title product m.p. 218° C.(dec)

## Example 62

9-p-Methoxybenzyl-6,8-difluoro-1,2,3,4-tetrahydrocarbazol-1-yl-acetic acid

Following the procedure of Example 45, but substituting p-methoxybenzyl chloride for o-chloro benzyl bromide as starting material, the title compound was obtained m.p. 149-151 °C.

The following example illustrates that the compounds of Formula I are useful for limiting cyclosporine induced nephrotoxicity.

## Example 63

All experiments were carried out using female spontaneously hypertensive (SHR) rats, weight range 160-200 gm, obtained from Taconic Farms. Animals were dosed for 14 days with either olive oil (0.1 ml/100 gm) or olive oil together with cyclosporine A (Sandimmune oral solution, 100 mg/ml) 25 mg/kg) together with either antagonist vehicle control (either 0.1% $NaHCO_3$, 0.9% saline or 1% Methocel; all at 0.1 ml/100 gm) or a solution or suspension of the thromboxane antagonist in the appropriate vehicle. Three thromboxane antagonists have been studied. These were the compounds of Examples 1, 37 and 50, hereinafter referred to herein as compounds 1, 37 and 50, respectively. Thromboxane antagonists have been administered as the sodium salt dissolved in saline, the sodium salt dissolved in 0.1% $NaHCO_3$ or the free acid suspended in 1% Methocel. The dose of cyclosporine A used produced a significant rise in serum blood urea nitrogen (BUN) after 14 days treatment indicative of abnormal renal function (serum BUN was measured by Lakeshore Diagnostic Laboratories using serum from blood samples obtained from the dorsal aorta 24 hours after the last dose of cyclosporine A). Higher doses of cyclosporine A (50 mm/kg) produced a significant morbidity during the treatment period.

In Table I, significant inhibition of the increase in serum BUN was obtained with a dose of compound 1. However, in a second experiment (Table 2) no significant inhibition was observed.

Table 3 shows the effects of Compound 37 administered as a suspension of the free acid, on cyclosporine A-induced rises in serum BUN and is a combination of two separate experiments. Both doses of Compound 37 (3 and 10 mg/kg/day) produced significant inhibition of the rise in serum BUN. Table 4 shows the effects of Compound 50 administered as a suspension of the free acid on cyclosporine A-induced rises in serum BUN. As with Compound 37 the compound was effective at both 3 and 10 mg/kg/day.

TABLE 1

| Effects of Compound 1 in saline on cyclosporine A-induced nephrotoxicity | | | |
|---|---|---|---|
| Experiment 1 | | | |
| Treatment | Serum BUN ± S.E.M. (mg/100 ml) | (n)** | P value compared* to cyclosporine A alone |
| Vehicles alone (saline & olive oil) | 23.6 ± 1.2 | (5) | <0.01 |
| Cyclosporine A 25 mg/kg/day | 30.5 ± 1.0 | (7) | - |
| Cyclosporine A + Compound 1 (3 mg/kg/day) | 24.6 ± 1.7 | (5) | <0.01 |

* Dunnet's multiple range analysis

** n = number of determinations

TABLE 2

| Effects of Compound 1 in saline on cyclosporine A-induced nephrotoxicity | | | |
|---|---|---|---|
| Combined experiments | | | |
| Treatment | Serum BUN ± S.E.M. (mg/100 ml) | (n)** | P value compared* to cyclosporine A alone |
| Vehicles alone (saline & olive oil) | 23.6 ± 1.1 | (10) | <0.01 |
| Cyclosporine A 25 mg/kg/day | 30.9 ± 0.8 | (10) | - |
| Cyclosporine A + Compound 1 (3 mg/kg/day) | 30.3 ± 2.0 | (10) | n.s. |

* Dunnet's multiple range analysis
** n = number of determinations

TABLE 3

| Effects of Compound 37 administered as a suspension in 1% Methocel on cyclosporine A-induced nephrotoxicity | | | |
|---|---|---|---|
| Treatment | Serum BUN ± S.E.M. (mg/100 ml) | (n)*** | P value compared to cyclosporine A alone* |
| Vehicles alone (1% Methocel & olive oil) | 22.8 ± 1.1 | (10) | <0.01 |
| Cyclosporine A 25 mg/kg/day | 28.8 ± 0.9 | (10) | - |
| Cyclosporine A + Compound 37 (3 mg/kg/day) | 23.2 ± 0.9 | (10) | <0.01 |
| Cyclosporine A + Compound 37 (10 mg/kg/day) | 24.0 ± 1.1 | (10) | <0.01 |

* Dunnet's multiple range analysis
** Results obtained from 2 separate experiments
*** n = number of determinations

TABLE 4

| Effects of Compound 50 administered as a suspension in 1% Methocel on cyclosporine A-induced nephrotoxicity | | | |
|---|---|---|---|
| Treatment | Serum BUN ± S.E.M. (mg/100 ml) | (n)** | P value compared to cyclosporine A alone* |
| Vehicles alone (1% Methocel & olive oil) | 22.3 ± 0.9 | (8) | <0.01 |
| Cyclosporine A 25 mg/kg/day | 26.4 ± 0.9 | (8) | - |
| Cyclosporine A + Compound 50 (3 mg/kg/day) | 23.5 ± 1.0 | (8) | <0.05 |
| Cyclosporine A + Compound 50 (10 mg/kg/day) | 21.9 ± 0.8 | (8) | <0.01 |

* Kruskal-Wallis multiple comparison test
** n = number of determinations

## EXAMPLE 64

Experiments were carried out using groups of 6 female spontaneously hypertensive (SHR) rats, weight range 166-194 gm, obtained form Taconic Farms. Animals were dosed for 5 weeks with either olive oil (0.1 ml/100 gm) or olive oil together with cyclosporine A (Sadimmune oral solution, 100 mg/ml) (25 mg/kg) together with either antagonist vehicle control 1% methocel; 0.1 ml/100 gm) or a suspension of Compound

63 in 1% methocel. At weekly intervals form 2 weeks onwards, animals were placed in individual metabolism cages and 24 hr urin samples collected, the volume measured and the creatinine levels determined (Lakeshore Diagnostic Laboratories). Creatinine clearance per rat was calculated from the formula:

$$\text{Creatinine clearance} = \frac{U_{Cr} \times V}{P_{Cr}}$$

where $U_{Cr}$ = concentration of creatine in urine
$P_{Cr}$ = concentration of creatinine in plasma
V = volume of urine flow in ml/min

After removal from the metabolism cage, a blood sample was taken from each rat for determination of serum BUN and creatinine (Lakeshore Diagnostic Laboratories). In a preliminary study, groups of 8 rats were dosed as above for 2 weeks only and serum samples removed for determination of BUN and creatinine as above.

In a pilot study, animals were treated with either vehicle controls, cyclosporine A alone (25 mg/kg/day), cyclosporine A and Compound 63 (3 mg/kg/day) or cyclosporine A and Compound 63 (10 mg/kg/day). The results are shown in Table 1. Treatment with cyclosporine A (25 mg/kg/day) produced significant increases in serum BUN and creatinine as compared to vehicle control. Treatment with compound 63 (3 or 10 mg/kg/day) significantly reduced the increase in serum BUN to control levels (Table 2) and the lower dose of Compound 63 significantly reduced the increase in serum creatinine (the reuslts with the higher dose failed to reach statistical significance for this parameter p = 0.07).

As no significant differences were seen between tge two doses of Compound 63, the higher dose (10 mg/kg/day) was used in a 5 week study in which the additional parameter of creatine clearance was measured. In addition, a second control group was added in which Compound 63 was administered together with vehicle for cyclosporine A to check that the drug alone was not significantly altering renal function. During the 5 week period, all groups showed the same weight gain, the changes in weight being 24.3 ± 1.9, 23.3 ± 2.6, 22.0 ± 1.9 and 25.0 ± 2.0 for the groups treated with vehicle controls, cyclosporine A (25 mg/kg/day), Compound 63 (10 mg/kg.day) and cyclosporine A and Compound 63 respectively (results are shown as means ± S.E.M., n = 6 and are expressed as change in body weight in gm). The results of the study are shown in Tables 3.5. Administration of Compound 63 alone produced no significant changes in any of the measured parameters as compared to the group treated with vehicle controls. The administration of cyclosporine A alone produced significant elevations in serum BUN at weeks 2, 3 and 5, no significant changes in seum cratinine and significant decreases in creatinine clearance at weeks 3 and 5. These changes were prevented by cotreatment with Compound 63 and the groups treated with Compound 63 and cyclosporine A as compared to those treated with cyclosporine A alone were significantly different with regard to serum BUN at weeks 3 and 5, serum creatinine at weeks 3 and creatinine clearance at week 3 (week 5 just failed to reach statistical significance, p = 0.07).

The results indicate that a tromboxane antagonist, Compound 63, inhibits cyclosporine A-induced nephrotoxicity.

Table 1

| Effects of Compound 63 on Cyclosporine A-induced Changes in Serum BUN | | | | |
|---|---|---|---|---|
| Parameter (mg/dl) | Control | Cyclosporine A (25 mg/kg/day) | Cyclosporine A and Compound 63 (3 mg/kg/day) | Cyclosporine A and Compound 63 (10 mg/kg/day) |
| Serum BUN | 22.2 ± 0.9** | 24.5 ± 0.9 | 20.8 ± 1.0* | 22.2 ± 0.7** |
| Serum Creatinine | 0.67 ± 0.02* | 0.73 ± 0.01 | 0.64 ± 0.02** | 0.65 ± 0.04 |
| Results are shown as means ± S.E.M. (n = 8) | | | | |

*P < 0.05 compared to group treated with cyclosporine A alone.
**P < 0.01 compared to group treated with cyclosporine A alone.

Table 2

| Mean Serum BUN Values | | | | |
|---|---|---|---|---|
| Week of Treatment | Control | Cyclosporine A (25 mg/kg/day) | Compound 63 alone 10 mg/kg/day | Compound 63 and Cyclosporine A |
| 2 | 23.4 ± 1.3 | 28.2 ± 1.4* | 23.5 ± 0.6 | 26.4 ± 0.9 |
| 3 | 24.5 ± 1.4 | 27.2 ± 1.5* | 21.5 ± 2.2 | 22.3 ± 1.7 |
| 4 | 22.8 ± 1.3 | 26.7 ± 2.6 | 21.3 ± 0.7 | 24.0 ± 1.4 |
| 5 | 24.3 ± 0.9 | 28.8 ± 1.3* | 25.7 ± 1.0 | 21.0 ± 1.1** |
| Results are expressed as mg/dl and are shown as means ± S.E.M. | | | | |

*P < 0.05 when compared to control

**P < 0.05 when compared to cyclosporine A-treated group.

Table 3

Mean Serum Creatinine Levels

| Week of Treatment | Control | Cyclosporine A (25 mg/kg/day) | Compound 63 (10 mg/kg/day) | Compound 63 and Cyclosporine |
|---|---|---|---|---|
| 2 | 0.49 ± 0.01 | 0.50 ± 0.04 | 0.54 ± 0.03 | 0.51 ± 0.03 |
| 3 | 0.50 ± 0.02 | 0.48 ± 0.02 | 0.46 ± 0.01 | 0.44 ± 0.01* |
| 4 | 0.56 ± 0.03 | 0.57 ± 0.2 | 0.55 ± 0.01 | 0.54 ± 0.02 |
| 5 | 0.41 ± 0.02 | 0.43 ± 0.02 | 0.42 ± 0.01 | 0.42 ± 0.01 |

Results are expressed as mg/d and are shown as means ± S.E.M. There are no significant differences between any of the groups apart from week 3 where the cyclosporine and Compound 63 treated group was significantly lower than the group treated with cyclosporine A alone (P = 0.02)

Table 4

Mean Creatinine Clearance Rates

| Week of Treatment | Control | Cyclosporine A (25 mg/kg/day) | Compound 63 alone (10 mg/kg/day) | Compound 63 and Cyclosporine A |
|---|---|---|---|---|
| 2 | 1.11 ± 0.10 | 1.02 ± 0.10 | 1.19 ± 0.08 | 1.20 ± 0.12 |
| 3 | 1.48 ± 0.17 | 1.09 ± 0.10* | 1.32 ± 0.07 | 1.42 ± 0.13** |
| 4 | 1.02 ± 0.09 | 0.89 ± 0.10 | 1.01 ± 0.07 | 0.91 ± 0.09 |
| 5 | 1.57 ± 0.20 | 1.10 ± 0.18$^{+}$ | 1.68 ± 0.08 | 1.44 ± 0.13$^{++}$ |

Results are expressed as ml/min and are shown as means ± S.E.M.

*$P < 0.05$, $^{+}P = 0.05$ when compared to control

**$P < 0.05$, $^{++}P = 0.07$ when compared to cyclosporin A alone.

## Claims

1. The use of a compound of Formula I:

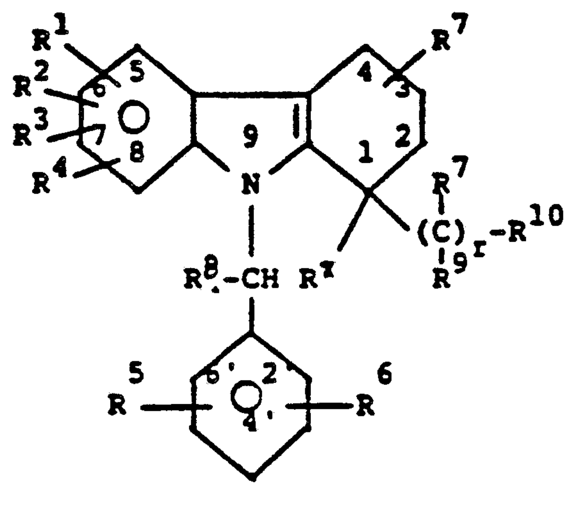

(I)

wherein:

$R^1$, $R^2$, $R^3$, $R^4$, $R^5$ and $R^6$ are each independently selected from:

(1) hydrogen;

(2) alkyl having 1 to 6 carbon atoms;

(3) alkenyl having 2 to 6 carbon atoms;

(4) $-(CH_2)_nM$

wherein n is 0 to 3 and M is

a) $OR^{13}$;
b) halogen;
c) $CF_3$;
d) $SR^{13}$;
e) phenyl or substituted phenyl wherein substituted phenyl is as defined below in the definition of $R^{13}$;
f) $COOR^{14}$;
g) $\overset{O}{\overset{\parallel}{C}}-R^{15}$;
h) tetrazole;
i) $-NH-\overset{O}{\overset{\parallel}{C}}-R^{16}$ wherein $R^{16}$ is $C_1$ to $C_6$ alkyl, benzyl or phenyl;
j) $-NR^{14}R^{14}$;
k) $-NHSO_2R^{17}$ wherein $R^{17}$ is $C_1$ to $C_6$ alkyl, 4-methylphenyl, phenyl, or $CF_3$;
l) $-\overset{O}{\overset{\parallel}{C}}-CH_2OH$;
m) $-SOR^{13}$;
n) $-CONR^{14}R^{14}$;
o) $-SO_2NR^{14}R^{14}$;
p) $-SO_2R^{13}$;
q) $NO_2$;
r) $O-\overset{O}{\overset{\parallel}{C}}-R^{15}$;
s) $O-\overset{O}{\overset{\parallel}{C}}-NR^{14}R^{14}$;
t) $O-\overset{O}{\overset{\parallel}{C}}-OR^{16}$;
u) CN;
v) $N_3$;

$R^7$ is H or alkyl of 1 to 6 carbons;
$R^8$ is or alkyl of 1 to 6 carbon atoms;
each $R^9$ is independently H, OH, $C_1$ to $C_4$-O-alkyl or alkyl of 1 to 4 carbons;
$R^{10}$ is COOH; $CH_2OH$; CHO; tetrazole;
$NHSO_2R^{11}$ wherein $R^{11}$ is OH, alkyl or alkoxy of 1 to 6 carbons, perhaloalkyl of 1 to 6 carbons, phenyl or phenyl substituted by alkyl or alkoxy groups of 1 to 3 carbons, halogen, hydroxy, COOH, CN, formyl or acyl to 1 to 6 carbons; $CONHSO_2R^{11}$; hydroxymethylketone; CN; or $CON(R^9)_2$;
r is 1 to 6;
each $R^{13}$ independently is H; $C_1$ to $C_6$ alkyl; benzyl; phenyl or substituted phenyl wherein the substituents are $C_1$ to $C_3$ alkyl, halogen, CN, $CF_3$, $COOR^{14}$, $CH_2COOR^{14}$, $C_1$ to $C_3$ alkoxy, or $C_1$ to $C_4$ perfluoroalkyl;
each $R^{14}$ is independently H, phenyl, benzyl or $C_1$ to $C_6$ alkyl; and,
each $R^{15}$ independently is H, $(CH_2)_mCOOR^{14}$ wherein m is 0 to 4, $C_1$ to $C_6$ alkyl, $CF_3$, phenyl, or substituted phenyl wherein substituted phenyl is as defined above in the definition of $R^{13}$;
or a pharmaceutically acceptable salt thereof; for the manufacture of a medicament for limiting cyclosporine induced nephrotoxicity.

2. The use as claimed in Claim 1, wherein:
$R^1$, $R^2$, $R^3$, $R^4$, $R^5$ and $R^6$ are each independently selected from:
(1) hydrogen;
(2) alkyl having 1 to 6 carbon atoms;
(3) alkenyl having 2 to 6 carbon atoms;
(4) $-(CH_2)_nM$
wherein n is 0 or 1 and M is as defined previously for Claim 1;
$R^{10}$ is COOH; $CH_2OH$; CHO; tetrazole; $CONHSO_2R^{11}$ wherein $R^{11}$ is OH, alkyl or alkoxy of 1 to 6 carbons, perhaloalkyl of 1 to 6 carbons, phenyl or phenyl substituted by alkyl or alkoxy groups of 1 to 3 carbons, halogen, hydroxy, COOH, CN, formyl or acyl to 1 to 6 carbons; hydroxymethylketone; CN; or $CON(R^9)_2$;
r is 1 to 6.

3. The use as claimed in Claim 2, wherein:
$R^1$, $R^2$, $R^3$, $R^4$, $R^5$ and $R^6$ are each independently selected from:
(1) hydrogen;
(2) alkyl having 1 to 6 carbon atoms;
(3) alkenyl having 2 to 6 carbon atoms;
(4) M wherein M is as defined initially for Claim 1;
$R^{10}$ is COOH; $CH_2OH$; CHO; tetrazole; hydroxymethylketone;
r is 1 or 2.

4. The use as claimed in Claim 3, wherein:
$R^1$, $R^2$, $R^3$, $R^4$, $R^5$ and $R^6$ are each independently selected from:
(1) hydrogen;
(2) alkyl having 1 to 6 carbon atoms;
(3) M wherein M is

a) $OR^{13}$;
b) halogen;
c) $CF_3$;
d) $SR^{13}$;
e) $COOR^{14}$;
f) $\overset{O}{\overset{\|}{C}}-R^{15}$;
g) tetrazole;
h) $-SOR^{13}$;
i) $-CONR^{14}R^{14}$;
j) $-SO_2NR^{14}R^{14}$;
k) $-SO_2R^{13}$;
l) $O-\overset{O}{\overset{\|}{C}}-R^{15}$;
m) CN;
n) $N_3$;

each $R^9$ is independently H, or alkyl of 1 to 4 carbons;
$R^{10}$ is COOH; or tetrazole;
r is 1.

5. The use as claimed in Claim 1 wherein the compound of Formula I is selected from:
9-p-chlorobenzyl-6-fluoro 1,2,3,4-tetrahydrocarbazol-1-yl-acetic acid;
3-[9-p-chlorobenzyl-6-fluoro-1,2,3,4 tetrahydrocarbazol-1-yl]-propanoic acid;
3-[9-p-chlorobenzyl-6-methoxy-1,2,3,4-tetrahydrocarbazol-1-yl]-propanoic acid;
9-p-chlorobenzyl--methoxy-1,2,3,4-tetrahydrocarbazol-1-yl-acetic acid;
2-[9-p-chlorobenzyl-6-fluoro-1,2,3,4-tetrahydrocarbazol-1-yl]-1 ethanol;
3-[9-p-chlorobenzyl-6-methoxy-1,2,3,4-tetrahydrocarbazol-1-yl]-propanol;
(-)9-p-chlorobenzyl-6-fluoro-1,2,3,4-tetrahydrocarbazol-1-yl-acetic acid;
(+)9-p-chlorobenzyl-6-fluoro-1,2,3,4-tetrahydrocarbazol-1-yl-acetic acid;
9-benzyl-6-fluoro-1,2,3,4 tetrahydrocarbazol-1-yl-acetic acid;
9-p-methoxybenzyl-6-fluoro-1,2,3,4-tetrahydrocarbazol-1-yl-acetic acid;
9-(3,4-dichloro)benzyl-6-fluoro-1,2,3,4-tetrahydrocarbazol-1-yl-acetic acid;
9-[1-(1-phenyl)ethyl]-6-fluoro-1,2,3,4-tetrahydrocarbazol-1-yl-acetic acid;
9-p-chlorobenzyl-1,2,3,4-tetrahydrocarbazol-1-yl-acetic acid;
9-p-chlorobenzyl-6-chloro-1,2,3,4-tetrahydrocarbazol-1-yl-acetic acid;
9-p-chlorobenzyl-8-methyl-1,2,3,4-tetrahydrocarbazol-1-yl-acetic acid;
6-bromo-9-p-chlorobenzyl-1,2,3,4-tetrahydrocarbazol-1-yl-acetic acid;
9-p-chlorobenzyl-6-methyl-1,2,3,4-tetrahydrocarbazol-1-yl-acetic acid;
2-[9-p-chlorobenzyl-6-fluoro-1,2,3,4-tetrahydrocarbazol-1-yl-]propanoic acid;
9-p-chlorobenzyl-8-fluoro-1,2,3,4-tetrahydrocarbazol-1-yl-acetic acid;
3-[($\alpha$ and $\beta$)-t-butyl-6-fluoro-9-(p-chlorobenzyl)-1,2,3,4-tetrahydrocarbazol-1-yl]acetic acid;
9-p-chlorobenzyl-5-fluoro-1,2,3,4-tetrahydrocarbazol-1-yl-acetic acid;
9-p-chlorobenzyl-7-fluoro-1,2,3,4-tetrahydocarbazol-1-yl-acetic acid;
9-p-chlorobenzyl-5,7-dichloro-1,2,3,4-tetrahydrocarbazol-1-yl-acetic acid;
9-p-chlorobenzyl-6,8-dichloro-1,2,3,4-tetrahydrocarbazol-1-yl-acetic acid.

9-p-chlorobenzyl-6-isopropyl-1,2,3,4-tetrahydrocarbazol-1-yl-acetic acid;
9-p-chlorobenzyl-6-tert-butyl-1,2,3,4-tetrahydrocarbazol-1-yl-acetic acid;
9-p-chlorobenzyl-6-trifluoromethyl-1,2,3,4-tetrahydrocarbazol-1-yl-acetic acid;
9-p-chlorobenzyl-6-methylthio-1,2,3,4-tetrahydrocarbazol-1-yl-acetic acid,
9-p-chlorobenzyl-6-methylsulfinyl-1,2,3,4-tetrahydrocarbazol-1-yl-acetic acid,
9-p-chlorobenzyl-6-methylsulfonyl-1,2,3,4-tetrahydrocarbazol-1-yl-acetic acid,
9-p-chlorobenzyl-8-isopropyl-1,2,3,4-tetrahydrocarbazol-1-yl-acetic acid;
9-p-chlorobenzyl-8-methylthio-1,2,3,4-tetrahydrocarbazol-1-yl-acetic acid;
9-p-chlorobenzyl-8-methylsulfinyl-1,2,3,4-tetrahydrocarbazol-1-yl-acetic acid;
9-p-chlorobenzyl-6-fluoro-3-methyl-1,2,3,4-tetrahydrocarbazol-1-yl-acetic acid;
9-p-chlorobenzyl-6,8-difluoro-1,2,3,4-tetrahydrocarbazol-1-yl-acetic acid;
9-p-chlorobenzyl-6,8-dimethyl-1,2,3,4-tetrahydrocarbazol-1-yl-acetic acid;
9-p-chlorobenzyl-6-methoxy-8-methyl-1,2,3,4-tetrahydrocarbazol-1-yl-acetic acid;
(-)-9-p-chlorobenzyl-6,8-difluoro-1,2,3,4-tetrahydrocarbazol-1-yl-acetic acid;
(+)-9-p-chlorobenzyl-6,8-difluoro-1,2,3,4-tetrahydrocarbazol-1-yl-acetic acid; (-)-9-p-chlorobenzyl-8-methyl-1,2,3,4-tetrahydrocarbazol-1-yl-acetic acid;
(+)-9-p-chlorobenzyl-8-methyl-1,2,3,4-tetrahydrocarbazol-1-yl-acetic acid;
(-)-9-p-chlorobenzyl-8-fluoro-1,2,3,4-tetrahydrocarbazol-1-yl acetic acid;
(+)-9-p-chlorobenzyl-8-fluoro-1,2,3,4-tetrahydrocarbazol-1-yl-acetic acid;
2-(9-p-chlorobenzyl-6,8-difluoro-1,2,3,4-tetrahydrocarbazol-1-yl)ethanol;
(-) or (+) 2-(9-p-chlorobenzyl-6,8-difluoro-1,2,3,4-tetrahydrocarbazol-1-yl)ethanol;
9-o-Chlorobenzyl-6,8-difluoro-1,2,3,4-tetrahydrocarbazol-1-yl-acetic acid;
9-(2,4-Dichlorobenzyl)-6,8-difluoro-1,2,3,4-tetrahydrocarbazol-1-yl acetic acid;
9-p-Methylthiobenzyl-6,8-difluoro-1,2,3,4-tetrahydrocarbazol-1-yl-acetic acid;
9-p-Methylsulfinylbenzyl-6,8-difluoro-1,2,3,4-tetrahydrocarbazol-1-yl-acetic acid;
9-p-Methylsulfonylbenzyl-6,8-difluoro-1,2,3,4-tetrahydrocarbazol-1-yl-acetic acid;
(-)9-p-Methylsulfonylbenzyl-6,8-difluoro-1,2,3,4-tetrahydrocarbazol-1-yl-acetic acid;
(+)9-p-Methylsulfonylbenzyl-6,8-difluoro-1,2,3,4-tetrahydrocarbazol-1-yl-acetic acid;
9-p-Trifluoromethylbenzyl-6,8-difluoro-1,2,3,4-tetrahydrocarbazol-1-yl acetic acid;
9-p-Fluorobenzyl-6,8-difluoro-1,2,3,4-tetrahydrocarbazol-1-yl-acetic acid;
9-m-Chlorobenzyl-6,8-difluoro-1,2,3,4-tetrahydrocarbazol-1-yl-acetic acid;
9-p-Carbomethoxybenzyl-6,8-difluoro-1,2,3,4-tetrahydrocarbazol-1-yl-acetic acid;
9-p-Dimethylcarboxamidobenzyl-6,8-difluoro-1,2,3,4-tetrahydrocarbazol-1-yl-acetic acid;
9-p-Acetylbenzyl-6,8-difluoro-1,2,3,4-tetrahydrocarbazol-1-yl-acetic acid;
9-p-Dimethylaminosulfonylbenzyl-6,8-difluoro-1,2,3,4-tetrahydrocarbazol-1-yl-acetic acid;
9-p-Acetamidobenzyl-6,8-difluoro-1,2,3,4-tetrahydrocarbazol-1-yl acetic acid;
9-p-Methylsulfonamidobenzyl-6,8-difluoro-1,2,3,4-tetrahydrocarbazol-1-yl-acetic acid;
9-p-Methylureidobenzyl-6,8-difluoro-1,2,3,4-tetrahydrocarbazol-1-yl-acetic acid and
9-p-Methoxybenzyl-6,8-difluoro-1,2,3,4-tetrahydrocarbazol-1-yl-acetic acid.

6. The use as claimed in Claim 1, wherein the compound of Formula I is selected from:
9-p-chlorobenzyl-6-fluoro-1,2,3,4-tetrahydrocarbazol-1-yl-acetic acid;
9-p-chlorobenzyl-8-methyl-1,2,3,4-tetrahydrocarbazol-1-yl-acetic acid;
9-p-chlorobenzyl-8-fluoro-1,2,3,4-tetrahydrocarbazol-1-yl-acetic acid;
9-p-chlorobenzyl-6,8-difluoro-1,2,3,4-tetrahydrocarbazol-1-yl-acetic acid; and
9-p-Methylsulfonylbenzyl-6,8-difluoro-1,2,3,4-tetrahydrocarbazol-1-yl-acetic acid.

7. The use as claimed in Claim 1, wherein the compound of Formula I is a pure optical isomer.

8. The use as claimed in Claim 7, wherein the compound of Formula I is the (+)-isomer.

9. The use as claimed in Claim 7, wherein the compound of Formula I is the (-)-isomer.

10. The use as claimed in Claim 9, wherein the compound of Formula I is selected from:
(-)9-p-chlorobenzyl-6-fluoro-1,2,3,4-tetrahydrocarbazol-1-yl-acetic acid;
(-)-9-p-Chlorobenzyl-6,8-difluoro-1,2,3,4-tetrahydrocarbazol-1-yl-acetic acid;
(-)-9-p-Chlorobenzyl-8-methyl-1,2,3,4-tetrahydrocarbazol-1-yl-acetic acid;
(-)-9-p-Chlorobenzyl-8-fluoro-1,2,3,4-tetrahydrocarbazol-1-yl-acetic acid; and
(-)9-p-Methylsulfonylbenzyl-6,8-difluoro-1,2,3,4-tetrahydrocarbazol-1-yl-acetic acid.

European Patent Office

# EUROPEAN SEARCH REPORT

Application number

EP 88306563.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A2 - 0 201 165 (BEECHAM<br>* Abstract; page 1, lines 1-29 *<br>-- | 1-10 | A 61 K 31/40<br>//A 61 K 37/02<br>C 07 D 209/86<br>C 07 D 403/06 |
| A | PROGRESS IN DRUG RESEARCH, vol. 28, 1984<br>J.DREWS "The pharmacology of the immune system: Clinical and experimental perspectives"<br>pages 83-109<br>* Page 93, last passage *<br>-- | 1-10 | |
| A | PROGRESS IN DRUG RESEARCH, vol. 28, 1984<br>W.J.WECHTER et al. "Immunology in drug research"<br>pages 233-272<br>* Pages 248-250; especially page 249 *<br>-- | 1-10 | **TECHNICAL FIELDS SEARCHED (Int. Cl.4)** |
| P,A | EP - A1 - 0 234 708 (MERCK FROSST CANADA INC.)<br>* Abstract; page 2, line 28 - page 3, line 7; claims 1,8 *<br>-- | 1-10 | A 61 K 31/00<br>C 07 D 209/00<br>C 07 D 403/00<br>A 61 K 37/00 |
| A | DE - A - 2 337 340 (F.HOFFMANN-LA ROCHE & CO AG)<br>* Claims 1,18 *<br>-- | 1-10 | |
| A | DE - A - 2 263 682 (F.HOFFMANN-LA ROCHE & CO AG)<br>* Claim 8 * ---- | 1-10 | |

The present search report has been drawn up for all claims

| Place of search | Date of completion of the search | Examiner |
|---|---|---|
| VIENNA | 25-10-1988 | MAZZUCCO |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82